# EUROPEAN PATENT SPECIFICATION

(11) **EP 3 137 078 B1**
(45) Date of publication and mention of the grant of the patent: **20.03.2019**
(21) Application number: 15785846.5
(22) Date of filing: 01.05.2015
(51) Int. Cl.: A61K 31/4545, A61K 31/427, A61K 31/4178, A61K 31/46, A61K 31/451, A61P 31/12, A61K 45/06, A61K 31/426

(54) **TREATMENT OF HEPATITIS DELTA VIRUS INFECTION**
BEHANDLUNG VON HEPATITIS-DELTA-VIRUSINFEKTION
TRAITEMENT D'INFECTION DE VIRUS D'HÉPATITE DELTA

(30) Priority: 01.05.2014 US 201461987315 P; 02.09.2014 US 201462044766 P; 31.10.2014 US 201462073413 P; 22.04.2015 US 201562151349 P
(43) Date of publication of application: 08.03.2017
(73) Proprietor: Eiger Biopharmaceuticals, Inc., Palo Alto, CA 94306 (US)
(72) Inventor: CORY, David, Palo Alto, CA 94306 (US); CHOONG, Ingrid, Palo Alto, CA 94306 (US); GLENN, Jeffrey, S., Palo Alto, CA 94306 (US)
(74) Representative: Patent Boutique LLP
(86) International application number: PCT/US2015/028933
(87) International publication number: WO 2015/168648

(56) References cited:
- WO-A1-2005/117864
- WO-A2-2011/088126
- WO-A2-2011/088126
- US-A1- 2007 287 664
- US-A1- 2011 105 557
- BRUNO B. BORDIER ET AL: "In vivo antiviral efficacy of prenylation inhibitors against hepatitis delta virus", JOURNAL OF CLINICAL INVESTIGATION, vol. 112, no. 3, 1 August 2003 (2003-08-01), pages 407-414, XP055410998, US ISSN: 0021-9738, DOI: 10.1172/JCI200317704
- GHOSAL ET AL.: 'Identification of Human Liver Cytochrome P450 Enzymes Responsible For The Metabolism of Lonafamib (Sarasar' DRUG METABOLISM AND DISPOSITION, [Online] vol. 34, no. 4, pages 628 - 635, XP055235801 Retrieved from the Internet: <URL:httpJ/dmd.aspetjournals.org/content/34 14/628.full.pdf+html> [retrieved on 2015-06-22]

## Description

### FIELD OF INVENTION

The present disclosure provides methods for treating viral hepatitis resulting from Hepatitis delta virus (HDV) infection, and so relates to the fields of chemistry, medicinal chemistry, medicine, molecular biology, and pharmacology.

### BACKGROUND OF THE INVENTION

Hepatitis delta virus (HDV) causes the most severe form of viral hepatitis, and there is no effective medical therapy (see Lau, 1999, Hepatology 30:546-549). HDV always presents as a co-infection with HBV, and a co-infected patient is much more likely to die of complications of viral infection than a patient infected with HBV alone. Currently available anti-HBV agents include the following nucleotide or nucleoside reverse transcriptase (RT) inhibitors: Lamivudine, Adefovir, Entecavir, Telbivudine, Clevudine, and Tenofovir. HBV/HDV co-infection may be treated with alpha interferon therapy or therapy with pegylated interferon alpha-2a (alone or in combination with one of the foregoing RT inhibitors).

The HDV large delta antigen protein contains a CXXX box rendering it a substrate for prenylation (see Zhang and Casey, 1996, Annu. Rev. Biochem. 65:241-269) by the prenyl lipid farnesyltransferase (see Glenn et al., 1992, Science 256:1331-1333, and Otto and Casey, 1996, J. Biol. Chem. 271:4569-4572). Farnesylation of proteins catalyzed by FTase is an essential step in processing of a variety of proteins and occurs by transfer of the farnesyl group of farnesyl pyrophosphate to a cysteine at the C-terminal tetrapeptide of a protein in a structural motif sometimes referred to as the CAAX box. Further post-translational modifications of a farnesylated protein, including proteolytic cleavage at the cysteine residue of the CAAX box and methylation of the cysteine carboxyl, generally follow farnesylation. Molecular genetic experiments demonstrated that specific mutation of the prenylation site in large delta antigen prevents both its prenylation and HDV particle formation (see Glenn et al., 1992, supra; also see Glenn et al., 1998 J. Virol. 72(11): 9303-9306; also see Bordier et al., 2002 J. Virol. 76(20): 10465-10472). Bordier et al. (J Clin Invest. 2003; 112(3); 407-414) showed that several farnesyl transferase inhibitors were effective at clearing HDV viremia in a mouse model of HDV infection. There continues to be an ongoing need for agents to treat HDV infection.

### BRIEF SUMMARY OF THE INVENTION

The invention provides lonafarnib and ritonavir for use in the treatment of hepatitis delta virus (HDV) infection in a human patient, wherein lonafarnib and ritonavir are administered orally to the patient as a co-therapy. The invention also provides lonafarnib for use in the treatment, by oral administration, of hepatitis delta virus (HDV) infection in a human patient, wherein the patient is also orally administered ritonavir as a co-therapy. This specification also discloses a method for treating HDV infection by oral administration of lonafarnib in combination with a CYP3A4 inhibitor (e.g., ritonavir or cobicistat). This specification also discloses a method for treating HDV infection by oral administration of lonafarnib at a dose of about 50 mg QD or BID, about 75 mg QD or BID, or about 100 mg QD in combination with ritonavir administration QD or BID administered at a therapeutically effective dose or with another CYP3A4 inhibitor administered BID or QD at a therapeutically effective dose. This specification also discloses a method for treating HDV infection by oral administration of at least about 50 mg QD or BID or at least about 100 mg QD or BID in combination with ritonavir administration QD or BID administered at a therapeutically effective dose or another CYP3A4 inhibitor administered BID or QD at a therapeutically effective dose.

In one embodiment as specified by the claims, the patient receiving lonafarnib-ritonavir co-therapy receives lonafarnib at a daily dose of 50 mg/day to 150 mg/day, for example 50 mg/day, 75 mg/day, 100 mg/day or 150 mg/day, and ritonavir at a daily dose of 100 mg/day - 200 mg/day, for example 100 mg/day or 200 mg/day.

In one instance, the patient receives a daily dose of 150 mg lonafarnib and 200 mg ritonavir. For example, the patient may receive 75 mg lonafarnib BID and 100 mg ritonavir BID.

In one instance, the patient receives a daily dose of 100 mg lonafarnib and 200 mg ritonavir. For example, in one embodiment as specified by the claims, the patient receives 50 mg lonafarnib BID and 100 mg ritonavir BID.

In one instance, the patient receives a daily dose of 150 mg lonafarnib and 100 mg ritonavir. For example, the patient may receive 75 mg lonafarnib BID and 100 mg ritonavir QD.

In one instance, the patient receives a daily dose of 75 mg lonafarnib and 100 mg ritonavir. For example, the patient may receive 75 mg lonafarnib QD and 100 mg ritonavir QD.

In one instance, the patient receives a daily dose of 50 mg lonafarnib and 100 mg ritonavir. For example, the patient may receive 50 mg lonafarnib QD and 100 mg ritonavir QD.

In one instance, the patient receives oral lonafarnib at a daily dose of 50 mg/day, 75 mg/day to 150 mg/day, administered BID or QD and oral ritonavir at a daily dose of 100 mg/day - 200 mg/day administered BID or QD, for example 100 mg/day or 200 mg/day, where the treatment results in a serum lonafarnib concentration greater than 2,000 ng/mL, preferably greater than 4,000 ng/mL, more preferably in the range of about 3,500 ng/mL to about 7,500 ng/mL.

In one instance, the patient receives oral lonafarnib at a daily dose of 75 mg/day to 150 mg/day, administered BID or QD, optionally with a boosting agent, where the treatment results in a serum lonafarnib concentration greater than 2,000 ng/mL, preferably greater than 4,000 ng/mL, more preferably in the range of about 3,500 ng/mL to about 7,500 ng/mL.

In some instances, lonafarnib and ritonavir or similar boosting agent are administered to the patient in a course of therapy extending at least 30 days, more often at least 60 days or at least 90 days, even more often at least 120 days, sometimes for at least 150 days, and sometimes for at least 180 days. In some instances, dosing will be discontinued after virus levels have decreased to below 3 log HDV RNA copies/mL (below 1,000 copies/mL) or below the level of detection for a period of time (such as 1 to 3 months or longer).

In one instance, prior to the initiation of oral administration of lonafarnib and ritonavir, patient is prophylactically treated with at least one, and typically a combination of at least two GI modifying agents (one or more of an anti-emetic agent, an anti-diarrheal, and an antacid).

In another instance, the GI modifying agents are administered at the same time as lonafarnib and ritonavir, and lonafarnib is administered as a delayed release formulation, and does not release until after the GI modifying agents begin take effect.

These and other disclosures are described in more detail below.

### BRIEF DESCRIPTION OF THE DRAWINGS

**FIGURE 1** graphically illustrates the time course of HDV RNA levels (copies/mL) of Patients 4, 5, and 6. See **EXAMPLE 1.**
**FIGURE 2** graphically illustrates HDV RNA viral load in patients relative to serum levels of lonafarnib, in patients treated with 100 mg lonafarnib BID for 28 days.
**FIGURE 3** graphically illustrates HDV RNA viral titers in human patients treated with lonafarnib in doses of either 200 mg BID or 300 mg BID for a period of 28 days. See Example 2.

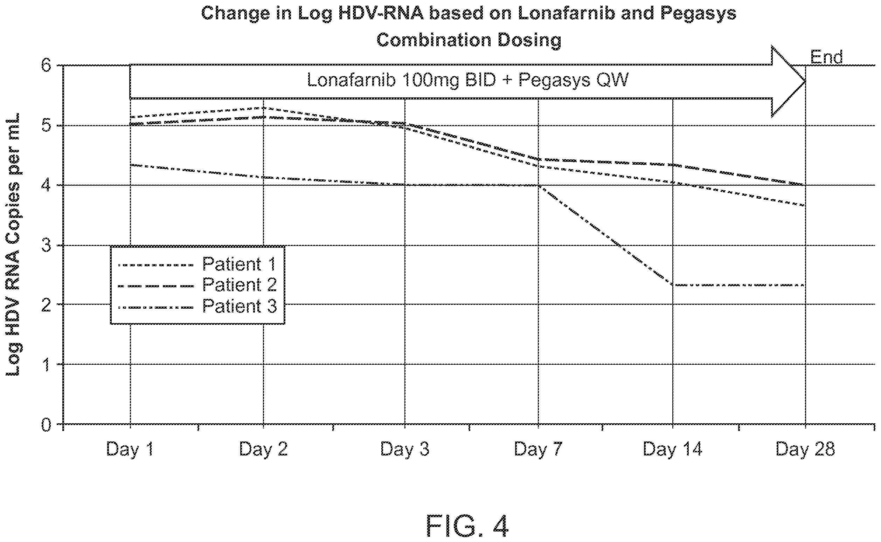

**FIGURE 4** graphically illustrates changes in HDV RNA viral titers in patients treated with lonafarnib and interferon at doses described in **EXAMPLE 3.**
**FIGURE 5** graphically illustrates HDV RNA viral titers in patients treated with lonafarnib at doses of 100 mg BID and ritonavir at 100 mg QD for a period of 28 days. See Example 4.
**FIGURE 6A** graphically illustrates changes in HDV RNA viral titers from a normalized baseline in patients treated with lonafarnib and ritonavir at doses described in **EXAMPLE 5** for a period of 28 days.
**FIGURE 6B** graphically illustrates changes in HDV RNA viral titers in patients treated with lonafarnib and ritonavir at doses described in Example 5 for a period of 56 days.
**FIGURE 6C** graphically illustrates changes in HDV RNA viral titers in patients treated with lonafarnib and ritonavir at doses described in Example 5 for a period of 84 days.
**FIGURE 7** graphically illustrates the inverse correlation between higher lonafarnib serum levels and HDV viral load.
**FIGURE 8** graphically illustrates a reduced correlation between lower lonafarnib serum levels and HDV viral load.

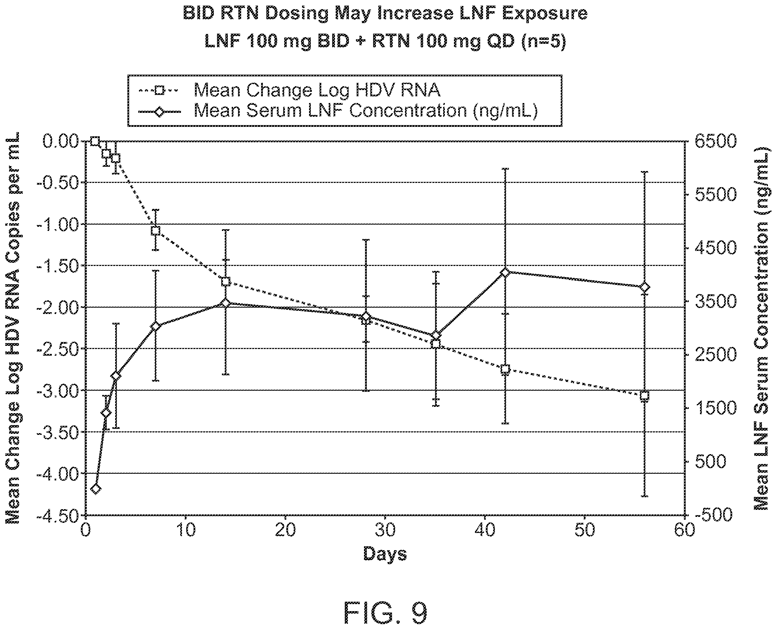

**FIGURE 9** graphically illustrates the relationship between lonafarnib serum concentration and change in viral load.
**FIGURE 10** graphically illustrates the changes in HDV RNA viral titers in patients treated with lonafarnib and a pegylated interferon or lonafarnib and ritonavir.

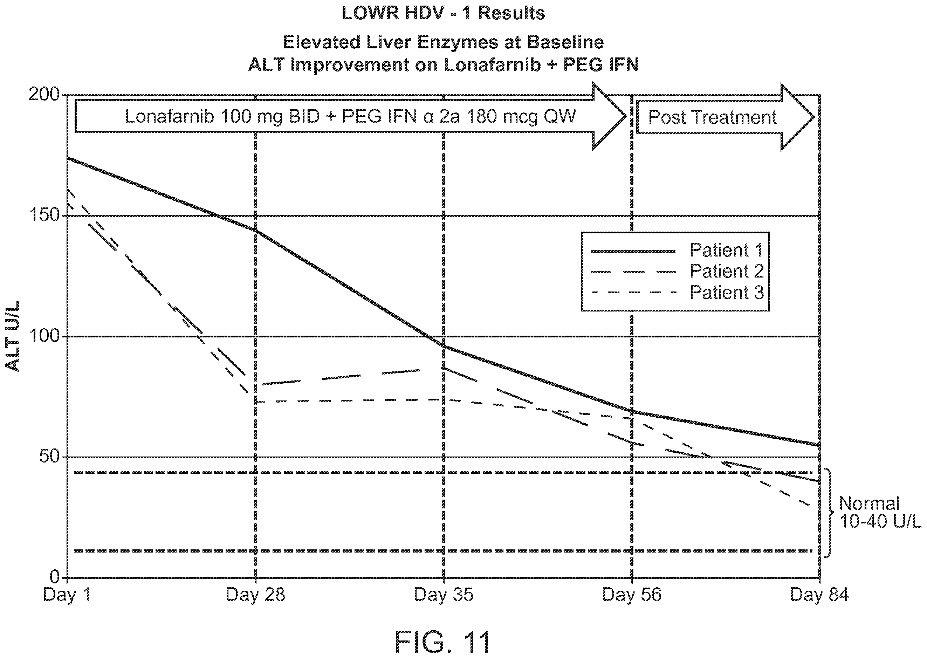

**FIGURE 11** graphically illustrates the changes in ALT values in patients treated with lonafarnib and a pegylated interferon.

### DETAILED DESCRIPTION OF THE INVENTION

The invention provides lonafarnib and ritonavir for use in the treatment of hepatitis delta virus (HDV) infection in a human patient, wherein lonafarnib and ritonavir are administered orally to the patient as a co-therapy. The invention also provides lonafarnib for use in the treatment, by oral administration, of hepatitis delta virus (HDV) infection in a human patient, wherein the patient is also orally administered ritonavir as a co-therapy. This detailed description of the invention is divided into sections for the convenience of the reader. As will be apparent to those of skill in the art upon reading this disclosure, each of the individual instances and embodiments described and illustrated herein has discrete components and features which may be readily separated from or combined with the features of any of the other several instances and embodiments (whether described in the same or different sections of this disclosure). Any recited method can be carried out in the order of events recited or in any other order that is logically possible. Embodiments and instances of the present disclosure will employ, unless otherwise indicated, techniques of synthetic organic chemistry, biochemistry, biology, molecular biology, recombinant DNA techniques, pharmacology, and the like, which are within the skill of the art. Such techniques are explained fully in the literature.

### I. Definitions

The terminology used herein is for the purpose of describing particular instances only, and is not intended to be limiting, because the scope of the present invention will be limited only by the appended claims. Unless defined otherwise, all technical and scientific terms used herein have the same meaning as commonly understood by one of ordinary skill in the art to which this invention belongs. In this specification and in the claims that follow, reference will be made to a number of terms that shall be defined to have the following meanings unless a contrary intention is apparent. In some cases, terms with commonly understood meanings are defined herein for clarity and/or for ready reference, and the inclusion of such definitions herein should not be construed as representing a substantial difference over the definition of the term as generally understood in the art.

Unless defined otherwise, all technical and scientific terms used herein have the same meanings as commonly understood by one of ordinary skill in the art to which this invention belongs. The preferred methods, devices, and materials are now described.

All numerical designations, e.g., pH, temperature, time, concentration, and molecular weight, including ranges, are approximations which are varied (+) or (-) by increments of 0.1 or 1.0, as appropriate. It is to be understood, although not always explicitly stated that all numerical designations are preceded by the term "about".

The singular forms "a," "an," and "the" include plural referents unless the context clearly dictates otherwise. Thus, for example, reference to "a compound" includes a plurality of compounds.

The term "administration" refers to introducing a compound, a composition, or an agent of the present disclosure into a host, such as a human. One preferred route of administration of the agents is oral administration. Other routes are intravenous administration and subcutaneous administration.

The term "comprising" is intended to mean that the compounds, compositions and methods include the recited elements, but not excluding others. "Consisting essentially of" when used to define compounds, compositions and methods, shall mean excluding other elements that would materially affect the basic and novel characteristics of the claimed invention. "Consisting of" shall mean excluding any element, step, or ingredient not specified in the claim. Instances defined by each of these transition terms are within the scope of this disclosure.

The term "Lonafarnib" or "EBP994", also known under the trade name Sarasar (Schering), refers to an FTase inhibitor 4(2[4-[(11R)-3,10-dibromo-8-chloro-6,11-dihydro-5Hbenzo[5,6]-cyclohepta[1,2b]pyridin-11yl]-piperidino]-2-oxoethyl]-1-piperidinecarboxamide) (also identified as Sch-66336 or SCH 66336) having the structure shown below:

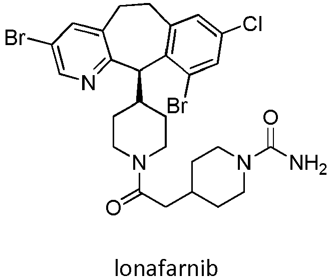

Lonafarnib is a crystalline solid with a melting point of approximately 200°C and is non-hygroscopic. Its molecular weight is 638.7. In the solid state, the compound is thermally stable. In solution, it is stable at neutral pH but will hydrolyze in acidic or basic conditions. It is a poorly water soluble tricyclic compound, which when formulated in crystalline forms results in low and variable bioavailability in animals. Considerable effort has been devoted to formulation development to improve the oral bioavailability. In addition to the drug substance, suitable pharmaceutical formulations of lonafarnib for administration in capsules contain povidone, poloxamer 188, croscarmellose sodium, silicon dioxide, and magnesium stearate. The product is formulated with a drug:povidone (1:1) coprecipitate to achieve optimal bioavailability. These are safe and well tested excipients that are commonly used in marketed products.

The term "HDV RNA viral load" or "viral load" of a human serum or plasma sample refers to the number of copies of human HDV RNA in a given amount of human serum or plasma sample. Currently, there is one commercially available test for the detection of HDV RNA (Quest Therapeutics), but no commercially available clinical tests for the quantitation of HDV RNA in clinical samples. However, several such assays reported in the literature (e.g., Kodani et al., 2013, J. Virol. Methods, 193(2), 531; and Karatayli et al., 2014, J. Clin. Virol, 60(1), 11) utilize a quantitative real-time reverse transcription-polymerase chain reaction (qRT-PCR) assay for quantification of HDV RNA in serum or plasma suitable for use in accordance with the methods of the invention. The amount of signal generated during the assay is proportional to the amount of HDV RNA in the sample. The signal from the test sample is compared to that of a dilution series of a quantified Hepatitis Delta RNA standard, and a copy number of genome copies is calculated.

The term "HDV infection" with respect to a human (host) refers to the fact that the host is suffering from HDV infection. Typically, an HDV infected human host will have a viral load of HDV RNA of at least about 2 log HDV RNA copies/mL of host serum or plasma or 102 copies of HDV-RNA/mL of host serum or plasma, often at least about 3 log HDV RNA copies/mL of host serum or plasma or 103 copies of HDV-RNA/mL of host serum or plasma, and, often, especially for patients not on any therapy, at least about 4 log HDV RNA copies/mL of host serum or plasma or 104 copies of HDV-RNA/mL of host serum or plasma, such as about 4 log HDV RNA copies/mL of host serum or plasma to 7 log HDV RNA copies/mL of host serum or plasma or 104-107 copies of HDV-RNA/mL of host serum or plasma.

The terms "patient", "host," or "subject," are used interchangeably and refer to a human infected with HDV, including patients previously infected with HDV in whom virus has cleared.

The term "pharmaceutical composition" is meant to encompass a composition suitable for administration to a subject. In general a "pharmaceutical composition" is sterile, and preferably free of contaminants that are capable of eliciting an undesirable response within the subject (e.g., the compound(s) in the pharmaceutical composition is pharmaceutical grade). Pharmaceutical compositions can be designed for administration to subjects or patients in need thereof via a number of different routes of administration including oral, intravenous, buccal, rectal, parenteral, intraperitoneal, intradermal, intracheal, intramuscular, subcutaneous, inhalational and the like.

The terms "pharmaceutically acceptable excipient," "pharmaceutically acceptable diluent," "pharmaceutically acceptable carrier," or "pharmaceutically acceptable adjuvant" means an excipient, diluent, carrier, and/or adjuvant that are useful in preparing a pharmaceutical composition that are generally safe, non-toxic and neither biologically nor otherwise undesirable, and include an excipient, diluent, carrier, and adjuvant that are acceptable for veterinary use and/or human pharmaceutical use. "A pharmaceutically acceptable excipient, diluent, carrier and/or adjuvant" as used in the specification and claims includes one and more such excipients, diluents, carriers, and adjuvants. A wide variety of pharmaceutically acceptable excipients, such as vehicles, adjuvants, carriers or diluents, and auxiliary substances, such as pH adjusting and buffering agents, tonicity adjusting agents, stabilizers, wetting agents and the like, are known in the art. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A.H. Kibbe et al., eds., 3rd ed. Amer. Pharmaceutical Assoc. For oral preparations, lonafarnib and/or ritonavir can be used alone or in pharmaceutical formulations disclosed herein comprising, or consisting essentially of, or consisting of lonafarnib in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The term "pharmaceutically acceptable salt" refers to those salts that retain the biological effectiveness and optionally other properties of the free bases and that are obtained by reaction with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid, malic acid, maleic acid, succinic acid, tartaric acid, citric acid, and the like. In the event that instances of the disclosed agents form salts, these salts are within the scope of the present disclosure. Reference to an agent of any of the formulas herein is understood to include reference to salts thereof, unless otherwise indicated.

The term "therapeutically effective amount" as used herein refers to that amount of an instance of the agent (which may be referred to as a compound, an inhibitory agent, and/or a drug) being administered that will treat to some extent a disease, disorder, or condition, e.g., relieve one or more of the symptoms of the disease, i.e., infection, being treated, and/or that amount that will prevent, to some extent, one or more of the symptoms of the disease, i.e., infection, that the subject being treated has or is at risk of developing.

The terms "treatment", "treating", and "treat" are defined as acting upon a disease, disorder, or condition with an agent to reduce or ameliorate the pharmacologic and/or physiologic effects of the disease, disorder, or condition and/or its symptoms. "Treatment," as used herein, covers any treatment of a disease in a human subject, and includes: (a) reducing the risk of occurrence of the disease in a subject determined to be predisposed to the disease but not yet diagnosed as infected with the disease, (b) impeding the development of the disease, and/or (c) relieving the disease, i.e., causing regression of the disease and/or relieving one or more disease symptoms. "Treatment" is also meant to encompass delivery of an inhibiting agent to provide a pharmacologic effect, even in the absence of a disease or condition. For example, "treatment" encompasses delivery of a disease or pathogen inhibiting agent that provides for enhanced or desirable effects in the subject (e.g., reduction of pathogen viral load, reduction of disease symptoms, etc.).

The term "unit dosage form," as used herein, refers to physically discrete units suitable as unitary dosages for human subjects, each unit containing a predetermined quantity of a compound (e.g., an anti-viral compound, as described herein) or compounds, calculated in an amount sufficient to produce the desired treatment effect in association with a pharmaceutically acceptable diluent, carrier or vehicle.

The term "oral dosage form," as used herein, refers to a dosage form that is that orally administered such as tablets, capsules, gel caps, syrups, elixirs, and suspensions. "Solid oral dosage forms" include tablets, capsules, caplets, and the like.

The term "oral unit dosage form," as used herein, refers to a unit dosage form that is that orally administered.

All deuterated analogs (a compound is a deuterated analog of another compound, the "parent compound", if it differs from the parent compound by only replacement of one or more hydrogen atoms with one or more deuterium atoms) of any active pharmaceutical ingredient described herein, including without limitation, lonafarnib, ritonavir, and cobicistat, are, for purposes of the present disclosure, encompassed by reference to the parent compound.

All stereoisomers of any agent described herein, including without limitation, lonafarnib, ritonavir, cobicistat, and any other active pharmaceutical agent described herein, such as those that may exist due to asymmetric carbons on the various substituents, including enantiomeric forms (which may exist even in the absence of asymmetric carbons) and diastereomeric forms, are contemplated within the scope of this disclosure. Individual stereoisomers of the compounds of the disclosure may, for example, be substantially free of other isomers, or may be admixed, for example, as racemates or with all other, or other selected, stereoisomers. The stereogenic centers of the compounds of the present disclosure can have the S or R configuration as defined by the IUPAC 1974 Recommendations.

The term "antacid" refers to agents that reduce, or reduce the effects of, gastric acid secretion, and includes H2-Receptor antagonists and proton pump inhibitors.

"H2-Receptor antagonists" are a class of drugs used to block the action of histamine on parietal cells (specifically the histamine H2 receptors) in the stomach, decreasing the production of acid by these cells. H2 antagonists are used in the treatment of dyspepsia.

"5-HT3 antagonists" are a class of drugs that act as receptor antagonists at the 5-HT3 receptor, a subtype of serotonin receptor found in several critical sites involved in emesis, including vagal afferents, the solitary tract nucleus (STN), and the area postrema itself. Serotonin is released by the enterochromaffin cells of the small intestine in response to chemotherapeutic agents and may stimulate vagal afferents (via 5-HT3 receptors) to initiate the vomiting reflex. The 5-HT3 receptor antagonists suppress vomiting and nausea by inhibiting serotonin binding to the 5-HT3 receptors. The highest concentration of 5-HT3 receptors in the central nervous system (CNS) are found in the STN and chemoreceptor trigger zone (CTZ), and 5-HT3 antagonists may also suppress vomiting and nausea by acting at these sites.

"NK1" is a G protein-coupled receptor located in the central and peripheral nervous system. This receptor has a dominant ligand known as Substance P (SP). SP is a neuropeptide, composed of 11 amino acids, which sends and receives impulses and messages from the brain. It is found in high concentrations in the vomiting center of the brain, and results in a vomiting reflux when activated. NK-1 receptor antagonists block signals given off by NK1 receptors.

"Proton pump inhibitors" are a class of antisecretory compounds that suppress gastric acid secretion by specific inhibition of the H+/K+ ATPase enzyme system at the secretory surface of the gastric parietal cell. Because this enzyme system is regarded as the acid (proton) pump within the gastric mucosa, inhibitors of this system have been characterized as a gastric acid-pump inhibitors in that they block the final step of acid production. This effect is dose-related and leads to inhibition of both basal and stimulated acid secretion irrespective of the stimulus.

"Anti-Diarrheal Agents" may be either of two types: Those that thicken the stool and those that slow intestinal spasms. Thickening mixtures (such as psyllium) absorb water. This helps bulk up the stool and make it more firm. Antispasmodic antidiarrheal products slow the spasms of the intestine by acting on the µ-opioid receptors in the myenteric plexus of the large intestine. By decreasing the activity of the myenteric plexus, which in turn decreases the tone of the longitudinal and circular smooth muscles of the intestinal wall, the amount of time substances stay in the intestine increases, allowing for more water to be absorbed out of the fecal matter. Anti-spasmodics also decrease colonic mass movements and suppress the gastrocolic reflex.

The term "GI intolerance" refers to any one of diarrhea, nausea, and vomiting individually, or combinations.

### II. Introduction

As specified by the claims, this invention relates to treatment of patients infected with hepatitis D virus (HDV) by coadministering the prenyltransferase inhibitor lonafarnib and the CYP3A4 inhibitor ritonavir. Although the use of lonafarnib to treat HDV infection was proposed in WO 2011/088126, the publication includes no *in vivo* efficacy data and described a broad range of doses (e.g., 25 to 300 mg per day). As is described hereinbelow, the present inventors have discovered that although administration of lonafarnib at 100 mg BID for 28 days reduced viral load, the reduction was not sufficient for development as a therapeutic. Higher doses of lonafarnib were poorly tolerated and resulted in an unacceptable level of adverse events. Thus, when administered at 200 mg BID, serum levels of lonafarnib dropped after about one month of treatment, mostly likely due to poor tolerability, poor compliance by patients, and loss of agents that pass through the GI tract. When administered at 300 mg BID, serum levels of lonafarnib were lower than expected, again, likely a result of poor tolerability. See, e.g., **TABLE 10,** below. Thus, while administration of lonafarnib at 100 mg BID was not sufficiently efficacious, higher doses were associated with significant GI-related adverse effects, rendering the treatment unsuitable as routine therapies for patients infected with HDV.

The present invention arises in part out of the discovery that administration of lonafarnib in combination with ritonavir ("lonafarnib-ritonavir cotherapy") according to dose schedules described herein is efficacious for treatment of HDV and results in superior outcomes compared to lonafarnib monotherapy. Surprisingly administration of 100 mg lonafarnib BID and 100 mg ritonavir Q.D resulted in *higher* serum concentrations of lonafarnib than observed with lonafarnib monotherapy measured after 56 days of treatment, but a *lower* frequence of adverse effects (see, e.g., **TABLES 9** and **10** below). Further, it has been discovered that lonafarnib-ritonavir cotherapy may be supplemented or combined with prophylactic administration of GI modifying agents (in particular prophylactic administration of one or more of an anti-emetic agent, an anti-diarrheal, and an antacid) to improve patient outcomes. Thus one embodiment of the invention, as specified by the claims, relates to lonafarnib-ritonavir cotherapy combined with prophylactic administration of a combination of GI modifying agents.

In one instance the patient receiving lonafarnib-ritonavir co-therapy receives lonafarnib at a daily dose of 75 - 150 mg per day, for example 75 mg per day, 100 mg per day, or 150 mg per day, and receives ritonavir at a daily dose of 100 mg - 200 mg per day, for example 100 mg per day or 200 mg per day. The aforementioned doses can be achieved by administering lonafarnib QD or BID and administering ritonavir QD or BID. In one approach, lonafarnib is administered BID and ritonavir is administered QD. In one approach both lonafarnib and ritonavir are administered BID. In one approach both lonafarnib and ritonavir are administered QD.

### III. HDV Treatment

The present specification provides methods for treating diseases relating to HDV infection, in which the HDV-infected patient is treated by oral administration of lonafarnib and ritonavir (which may be referred to as "lonafarnib administration," "lonafarnib-ritonavir cotherapy," and the like). Preferably lonafarnib and ritonavir are administered according to doses and dosing schedules described herein. In some instances, the HDV-infected patient receives prophylactic treatment with one, two, or three or more classes of gastrointestinal (GI) modifying agents. In some embodiments as specified by the claims, patients receiving lonafarnib-ritonavir cotherapy are also treated with interferon (e.g., interferon-alpha or interferon-lambda).

### Dosing schedules for lonafarnib-ritonavir cotherapy

Lonafarnib has been investigated for treatment of solid and hematological malignancies, Hutchinson-Gilford progeria syndrome, and chronic Hepatitis delta virus infection, but is not approved for any indication. The majority of reports concerning lonafarnib dosing are related to administration of lonafarnib to patients with cancer in combination with one or multiple antineoplastic agents.

Ritonavir (marketed under the trade name Norvir® by AbbVie, Inc.) has been administered as an antiretroviral agent, in combination with other antiviral agents, for treatment of HIV-1 infected individuals. *See* Miller et al., 2015, Infection and Drug Resistance, 8:19-29. For treatment of HIV-1 in adult patients, the recommended dosage of ritonavir is 600 mg twice daily by mouth to be taken with meals. *See* Norvir® package insert. Ritonavir has also been used as a pharmacologic enhancer or boosting agent. Pharmacokinetic "boosting" refers to the pharmacological enhancement of orally dosed drugs through the co-dosing with pharmacological enhancers which render these drugs more effective. Ritonavir has been used to boost the Cₘₐₓ of proteases used to treat HIV infection. The boosting effect of ritonavir results from several properties of the drug. Ritonavir inhibits two key stages of metabolism:

First, ritonavir inhibits first-pass metabolism during absorption. Enterocytes that line the intestine contain both CYP3A4, one of the key cytochrome P450 isoenzymes associated with drug metabolism, and P-glycoprotein, an efflux transporter that can effectively pump drugs out of the gut wall and back into the intestinal lumen. Ritonavir inhibits both of these proteins. Consequently, co-administration of ritonavir and a drug transported by P-glycoprotein and/or metabolized by enterocyte CYP3A4 may increase the Cmax of the co-administered drug. Second, ritonavir inhibits CYP3A4 in the liver, thereby maintaining a drug's plasma half-life.

Several factors make it impossible to predict, and difficult to determine, what an acceptable dose of ritonavir is for use as a boosting agent.

First, the boosting effects of ritonavir vary broadly and unpredictably depending on the primary (*i.e.,* co-administered) drug (PD). This is illustrated by the Norvir® package insert (available at the FDA website http://www.rxabbvie.com/pdf/norvirtab_pi.pdf) which shows that the effect of coadministration of ritonavir with a primary drug can range from a 350-fold increase AUC of the primary drug (Fluticasone propionate, delivered as aqueous nasal spray) to an 11-fold increase (sidenafil) to a 1.2-fold increase (trimethoprim). There is also great variance even within a single drug class. For example, in a meta-study of 17 dose-ranging pharmacokinetic trials of protease inhibitors, Hill et al. evaluated the ritonavir boosting effect at doses of 50-800 mg daily with seven protease inhibitors: amprenavir, atazanavir, darunavir, indinavir, lopinavir, saquinavir and tipranavir. Hill concluded that ritonavir has a dose-dependent boosting effect on Indinavir, tipranavir and lopinvir. For example, "there was a large rise in lopinavir Cmin when ritonavir dose was raised from 50 to 100 mg." However, the boosting effect of ritonavir on darunavir or saquinavir is not correlated with its dose. For example, a dose of ritonavir at 200 mg daily has the same effect as 100 mg daily on serum exposure for the drug ("with [a] dose of.. 200mg once daily,. the plasma exposure for darunavir for these doses was similar to ... 100 mg of ritonavir once daily"). Similarly, saquinavir trial results showed no significant correlation between the dose of ritonavir used and the achieved Cmax or Cmin of saquinavir.

In addition, the pharmacokinetics of ritonavir in patients with hepatitis is likely more unpredictable relative to other treatment populations. Li et al. reported that hepatic CYP3A4 expression is down-regulated in individuals with chronic HBV infection. Although the subpopulation of HBV-infected patients co-infected with HDV was not separately studied, it is likely CYP3A4 down-regulation occurs in HDV positive individuals. See Li et al., 2006, Zhonghua yi xue za zhi, 86:2703-2706.

It has also been reported that, ritonavir may inhibit P-glycoprotein found in peripheral blood lymphocytes. See Lucia et al., 2001, J Acquir Immune Defic Syndr. 27:321-30. If lonafarnib is a substrate of P-glycoprotein, the co-administration of ritonavir could cause less lonafarnib to be transported back out of the cell, thereby increasing the drug¹s intracellular half-life.

**Further,** the HDV patient subpopulation is characterized by higher levels of cirrhosis (which develops in about 60 to 70% of patients with chronic hepatitis D) than patients infected with HBV only. The pharmacokinetics of ritonavir in the HDV patient population will likely be more unpredictable relative to other populations.

The therapeutic effects of coadministration of lonafarnib and ritonavir to patients with chronic HDV were not known prior to the present invention, and nothing in the medical literature prior to the present invention described administration regimens (e.g., doses and dose scheduling) that are effective for treating patients with chronic HDV infection.

In addition, the side effect profiles of administration of lonafarnib and coadministration of lonafarnib and ritonavir have not been previously determined. Diarrhea, nausea, and vomiting are reported as side-effects of both lonafarnib administration (see, Schering IB) and ritonavir administration (see Norvir Package Insert). In cancer patients, a dose of 200 mg BID lonafarnib was characterized as "well tolerated." *See* Hanrahan et al., 2009, "A phase II study of Lonafarnib (SCH66336) in patients with chemorefractory, advanced squamous cell carcinoma of the head and neck," Am J Clin Oncol. 32:274-279 (describing lonafarnib therapy following platinum-based therapy for recurrent SCCHN) and List et al., 2002, Blood, 100:789A (a lonafarnib dose of 200 mg BID was well tolerated in patients with advanced hematologic malignancies). However, the side-effect profile of administration of therapeutically effective levels of lonafarnib to patients with chronic HDV infection was not known, and the side-effect profile of lonafarnib-ritonavir cotherapy was not known for any population.

### Effect of Lonafarnib Administration on HDV Infection

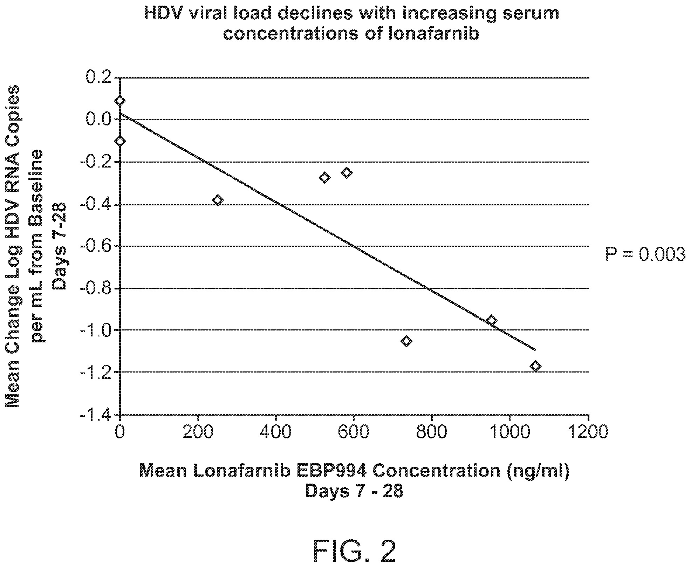

A cohort of patients with chronic delta hepatitis (HDV) received treatment with 100 mg lonafarnib BID for 28 days and showed a mean change in HDV RNA levels from baseline to nadir of -0.74 log HDV RNA copies/mL, compared to -0.24 log HDV RNA copies/mLin a patients receiving placebo. See **EXAMPLE 1,** below. Plasma levels of lonafarnib ranged between 200 ng/mL and 1,100 ng/mL during treatment and it was discovered in this study that subjects with higher plasma levels of lonafarnib experienced greater declines in HDV RNA titers during treatment. See **FIGURE 2****.** However, a more robust reduction in viral load is needed.

As described in **EXAMPLE 2,** below, administration of higher doses of lonafarnib to HDV-infected patients resulted in a more dramatic reduction of viral load. In patients receiving 200 mg BID lonafarnib for 28 days the mean change in viral load was -1.63 HDV RNA copies/mL. In patients receiving 300 mg BID lonafarnib 28 days the mean change in viral load was -2.00 HDV RNA copies/mL.

We conclude that daily administration of 200 mg BID lonafarnib provided superior viral load declines in HDV patients compared to a daily administration of 100 mg BID lonafarnib. However, administration of lonafarnib 200 mg/BID or 300 mg/BID resulted in significant adverse effects, which renders these dosage regimens unsuitable for long term therapy.

**TABLE 1**

| CHANGE IN VIRAL LOAD AFTER 28 DAYS IN PATIENTS RECEIVING LONAFARNIB OR LONAFARNIB WITH RITONAVIR | | |
|---|---|---|
| | Mean Log Change in Serum HDV RNA AT DAY 28 | N |
| Placebo | -0.24 | 4 |
| Lonafarnib 100 mg BID* | -0.74 | 6 |
| Lonafarnib 200 mg BID | -1.63 | 6 |
| Lonafarnib 300 mg BID | -2.00 | 3 |
| Lonafarnib 100 mg BID with Ritonavir 100 mg QD | -2.20 | 3 |

| | | |
|---|---|---|
| * Mean Plasma levels of lonafarnib ranged between 540 ng/mL and 890 ng/mL | | |

### Effect of Lonafarnib-Ritonavir Administration on HDV Infection

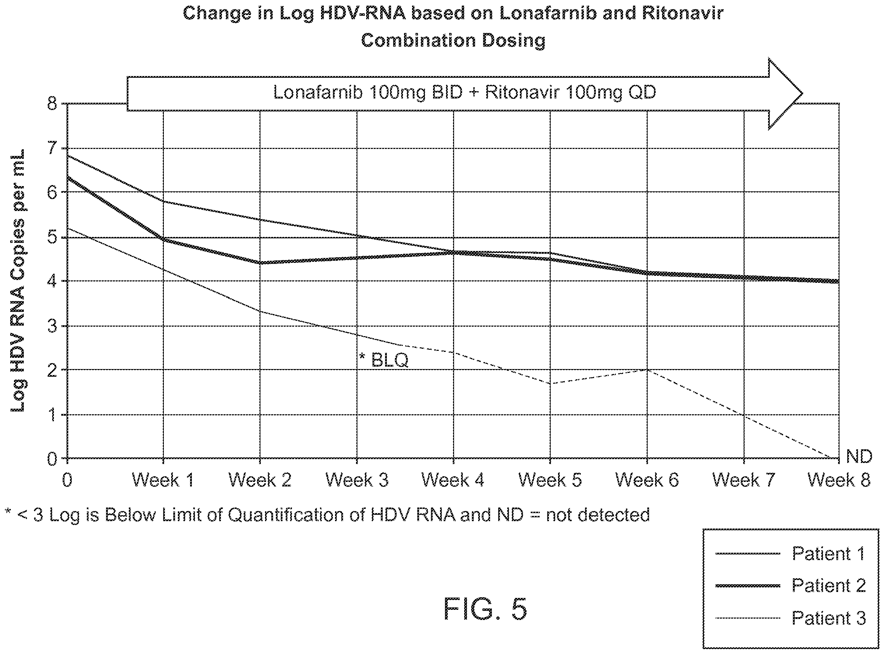

As illustrated in Examples 5-10, lonafarnib-ritonavir co-therapy reduced HDV viral load substantially, including, in one case, to undetectable levels at week 8. See **FIGURE 5****.** Accordingly, in various methods of this disclosure, lonafarnib and ritonavir each are administered orally on a continuous, daily basis, at least once per day (QD), and in various instances two times per day (BID).

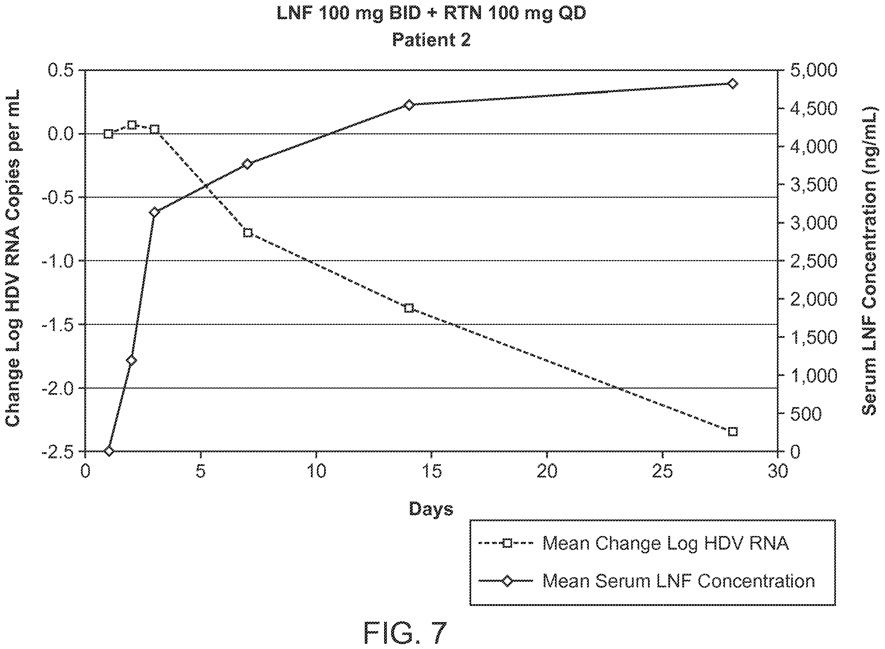

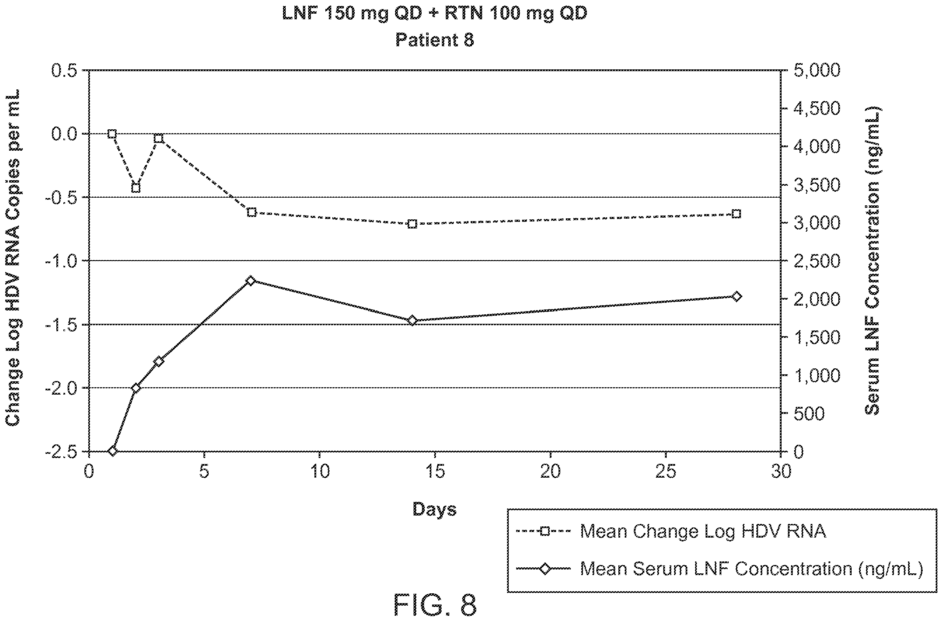

As shown in **FIGURE 2****,** HDV viral load declines with increasing serum concentrations of lonafarnib. The correlation between lonafarnib serum levels and viral load in patients receiving lonafarnib-ritonavir cotherapy is also illustrated by comparing the viral load of a patient who maintained a serum lonafarnib concentration in the range of about 3,500 to 5,000 ng/mL for about 21 days (see **FIGURE 7**) with the viral load of a patient who maintained a serum lonafarnib concentration in the range of about 1500 to 2500 ng/mL for about 21 days (see **FIGURES 7** **and** **8**). Also see **TABLE 9,** showing that the patient with the highest lonafarnib serum level after six weeks of co-therapy had the greatest decrease in viral load, and that patients with lonafarnib serum levels greater than 2,000 ng/mL had, in general, more dramatic reductions in viral load than patients with serum levels lower than 2,000 ng/mL, patient 4 being an exception to the trend.

In certain instances, lonafarnib and ritonavir are co-administered according to a schedule that results in the serum lonafarnib levels greater than 2,000 ng/mL, for example, greater than 4,000 ng/mL. In some instances, lonafarnib and ritonavir are co-administered according to a schedule that results in the serum lonafarnib levels are in the range of about 3,500 ng/mL to about 8,500 ng/mL (e.g., about 4,500 ng/mL to about 7,500 ng/mL , about 5,000 ng/mL to about 6,000 ng/mL, about 5,500 ng/mL to about 6,500 ng/mL, about 6,000 ng/mL to about 7,000 ng/mL, or about about 6,500 ng/mL to about 7,500 ng/mL) or about 5,000 ng/mL to about 7,000 ng/mL.

As used herein, a serum lonafarnib level or concentration can be measured from a serum sample obtained from a subject periodically (such as weekly, biweekly, monthly or according to other schedules) and the levels during intervening periods can be extrapolated. For example, if a measurement of 4,000 ng/mL is obtained at 4 weeks and a measurement of 6,000 ng/mL is obtained at 6 weeks, for purposes of this analysis it is concluded that the serum level during the intervening two weeks ranged between 4,000 and 6,000 ng/mL. In some instances the first measurement is made no earlier than one week after the initiation of oral therapy.

Serum levels of lonafarnib can be measured using art-known methods, including radioimmune assays, chromatographic assays, mass spectrometry and the like. In some instances of this disclosure, patient serum samples were extracted using a protein precipitation method (Acetonitrile). The samples were then loaded onto Waters CSH C18, 2.1 x 50 mm, 1.7 µm column for separation, followed by LC-MS/MS in positive ion mode for detection of lonafarnib. The assay range for lonafarnib was 1-2500 ng/mL.

### Exemplary Doses

Exemplary doses, for illustration and not for limitation, are provided in **TABLE 2.** Usually, lonafarnib and ritonavir are administered (e.g., self-administered by the patient) together at about the same time (e.g., simultaneously or within about 15 minutes of each other).

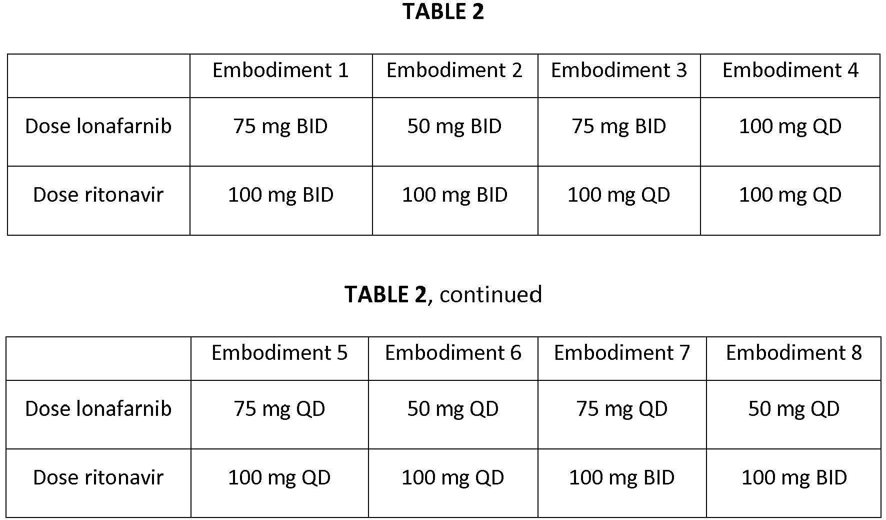

Each of Embodiments 1-8 in **TABLE 2** may be administered with prophylactic GI modifying agents (e.g., an anti-emetic agent, an anti-diarrheal agent, and an antacid). See **Section IV,** below.

In some instances, lonafarnib and ritonavir (or similar boosting agent, such as cobicistat) are administered to the patient, and both the ritonavir dose and the EBP994 dose are at least 100 mg QD for at least 30 days, usually at least about 60 or even 90 days or longer, including 6 months to a year or longer. In some instances, dosing will be discontinued after virus levels have decreased to undetectable levels for a period of time (such as 1 to 3 months or longer). In one approach suitable doses of lonafarnib/ritonavir include 100 mg QD/50 mg QD or BID, 100 mg QD/100 mg QD or BID, 100 mg QD/150 mg QD for least for 30 days, more often at least 60 days, and typically at least 90 days, or longer than 90 days. In one approach treatment of hepatitis delta virus (HDV) infection in a human involves administering a daily dose of about 100 mg QD lonafarnib, and a therapeutically effective amount of a CYP3A4 inhibitor (e.g., ritonavir or cobicistat) for at least about 30 days, thereby treating the HDV infection. In one approach ritonavir is dosed at 100 mg QD.

### Dose Escalation

In one instance the patient being treated for HDV infection receives an escalating dosage regimen of lonafarnib to increase the patient's tolerance to the drug and minimize side effects. The duration of the administration of each dosage in the escalating regimen is typically within 1-4 weeks, but may be adjusted (e.g., accelerated) by a clinician based on the patient's response. For example, without limitation, a patient may be given lonafarnib 50 mg BID for an initial two week periods, followed by 75 mg BID for a second two weeks period, and followed by 100 mg BID for a third two week period until a predetermined desired final dose is reached. Typically the escalating doses are co-administered with ritonavir at a suitable dose, for example, 100 mg QD or BID.

### Duration of treatment

Patients may receive lonafarnib-ritonavir cotherapy for a predetermined time, an indefinite time, or until an endpoint is reached. Treatment may be continued on a continuous daily basis for at least two to three months. Therapy is typically for at least 30 days, more often at least 60 days or at least 90 days, even more often at least 120 days, sometimes for at least 150 days, and sometimes for at least 180 days. In some instances, treatment is continued for at least six months to one year. In other instances, treatment is continued for the rest of the patient's life or until administration is no longer effective in maintaining the virus at a sufficiently low level to provide meaningful therapeutic benefit.

In some instances, therapy as disclosed herein is continued for a period of time until HDV RNA levels are below 3 log HDV RNA copies/mL (below 1,000 copies/mL), or sometimes until HDV RNA levels are below 2 log HDV RNA copies/mL (below 100 copies/mL) or below the level of detection. In some cases therapy may be continued for a period of time (such as 1 to 3 months or longer) after viral load has dropped to acceptably low levels (e.g., undetectable levels).

In some cases, therapy is continued until an "hepatitis flare" or "ALT flare" is observed in the patient. Hepatitis flares (or acute exacerbations) are an abrupt elevation of serum alanine aminotransferase (ALT) over fivefold the upper limit of normal, about 40 U/mL, observed in chronic hepatitis B virus (HBV) infection. HBV flares in HBV patients result from an HLA-I restricted, cytotoxic T lymphocyte (CTL)-mediated immune response against HBV and its downstream mechanisms. Higher ALT levels reflect a more robust immune clearance of HBV. See Liaw, 2003, "Hepatitis flares and hepatitis Be antigen seroconversion: implication in anti-hepatitis B virus therapy," J Gastroenterol Hepatol 18:246-52. Hepatitis flares have not previously been reported in response to anti-HDV treatment, but indications of flares have been observed in response to the lonafarnib-ritonavir cotherapy described herein. For example, two patients who received orally administered lonafarnib 100 mg BID and ritonavir 50 mg BID for 12 weeks exhibited ALT flares characterized by ALT levels 10-20 fold higher than a normal individual. ALT flares were also observed in some patients receiving lonafarnib 200 mg BID or 300 mg BID monotherapy. The observation of flares in HDV patients suggests that cotherapy with ritonavir and lonafarnib at dosages described herein may have an unprecedented therapeutic effect.

### IV. Prophylaxis With Gastrointestinal Modifying Therapies

As described in the Examples, HDV-infected patients receiving lonafarnib monotherapy and lonafarnib-ritonavir therapy experienced gastrointestinal (GI) side effects. Gastrointestinal (GI) side effects are not unexpected of compounds in the farnesyl transferase class. GI intolerance is also a known side-effect of ritonavir, which may be dosed at 1200 mg/day when used as a protease inhibitor. However, the severity and persistence of these symptoms in HBV patients (especially given the comparatively modest doses of lonafarnib and ritonavir) was not expected. Agents for treatment of gastrointestinal irritations include anti-emetics, antacids (H2-receptor antagonists and proton pump inhibitors) and anti-diarrheals. Exemplary agents (for illustration and not limitation) are listed in **TABLE 3.**

In accordance with the methods of the present specification, lonafarnib is used in combination with at least one, at least two, or at least three of an anti-emetic, an antacid (H2-receptor antagonist or proton pump inhibitor) and/or an anti-diarrheal to allow for continued compliance of patients while on lonafarnib therapy. In one instance, an anti-diarrheal agent is administered. In one instance, an anti-diarrheal agent and an antacid is administered. In one instance, an anti-diarrheal agent and an anti-emetic are administered. In one instance, an anti-diarrheal agent, an antacid and an anti-emetic are administered. In one instance, the anti-diarrheal agent is lomotil (atropine/diphenoxylate) and/or the antacid is famotidine and/or the antiemetic is ondansetron.

**TABLE 3**

| Exemplary GI Modifying Agents | |
|---|---|
| Class | Exemplary agents |
| Antiemetics | 5-HT₃ antagonists (such as ondansetron (Zofran®), tropisetron (Navoban®), granisetron (Kytril®), palonosetron (Aloxi®), and dolasetron (Anzemet®)) and NK1 receptor antagonists (such as aprepritant (Emend®), casopitant, and fosaprepitant (Emend® IV)). |
| Antacids | H2-receptor antagonists (such as ranitidine (Zantac®), famotidine (Pepcid®), cimetidine (Tagamet®) and nizatidine (Axid®) and Proton pump inhibitors (such as omeprazole (Prilosec®), omeprazole/sodium bicarbonate (Zegerid®), esomeprazole magnesium (Nexium®), esomeprazole strontium, lansoprazole (Prevacid®), dexlansoprazole (Dexilant®), rabeprazole, and pantoprazole sodium (Protonix®)). |
| Anti-diarrheals | atropine/diphenoxylate (Lomotil®, Lonox®), loperamide HCI (Imodium®), and bismuth subsalicylate (Kaopectate®, Pepto-Bismol®). |

In one approach, GI modifying therapies are administered on an as-needed basis (in response to symptoms). In one approach GI modifying therapies are administered prophylactically. As used herein in this context, "prophylactically" refers to administration to a patient in the absence of, or before development of, symptoms. Typically prophylactic treatment entails administration according to a fixed schedule (e.g., daily) during the course of lonafarnib treatment.

In one approach, prophylactic treatment comprises administration of ondansetron (anti-emetic), lomotil (atropine/diphenoxylate) (anti-diarrheal) and famotidine (antacid). For example, ondansetron may be administered 8 mg BID, lomotil (atropine/diphenoxylate) may be administered 5 mg QID or 5 mg BID, and famotidine may be administered 20 mg BID.

In one approach, the GI modifying agents are administered daily, prior to administration of lonafarnib. In one approach GI modifying agents are administered 30 minutes to two hours before administration of lonafarnib therapy

In one approach, the GI modifying agents are administered daily at the same time lonafarnib is administered, but lonafarnib (and optionally ritonavir) are administered as a delayed release formulation (e.g. comprising an enteric coating) so that the GI modifying agents begin to take effect prior to lonafarnib release.

Prophylactic administration of GI modifying agents is generally continued for the duration of lonafarnib therapy.

In one approach, prophylactic administration of GI modifying agents commences on the first day of lonafarnib administration. In another approach, administration of one or more of the GI modifying agents commences prior to initiation of oral lonafarnib-ritonavir therapy. For example, in one instance the patient takes ondansetron the day before the start of lonafarnib treatment. In one approach, one or more GI modifying agents are administered daily beginning more than one day before initiation of oral lonafarnib-ritonavir treatment.

In a preferred instance, a GI modifying agent is administered according to a BID or QD schedule.

### H2-Receptor Antagonists

In one instance of these GI modifying therapies, this GI modifying therapy is an H2-receptor antagonist. In one instance of these GI modifying therapies, ranitidine (Zantac®) is administered at a dose of 150 mg twice daily, up to 150 mg four times daily for the duration of lonafarnib therapy. In another instance of these GI modifying therapies, famotidine (Pepcid®) is administered at a dose of 40 mg once daily, up to 20 mg twice daily, up to 40 mg twice daily for the duration of lonafarnib therapy. In another instance of these GI modifying therapies, cimetidine (Tagamet®) is administered at dose of 400 mg once daily, up to 800 mg once daily, up to 1600 mg once daily, up to 800 mg twice daily, up to 300 mg four times daily, up to 400 mg four times daily, up to 600 mg four times daily for the duration of lonafarnib therapy. In another instance of these GI modifying therapies, nizatidine (Axid) is administered at dose of 150 mg once daily, up to 300 mg once daily, up to 150 mg twice daily for the duration of lonafarnib therapy.

### 5-HT₃ Antagonists

In one instance of these GI modifying therapies, this therapy is a 5-HT₃ receptor antagonist. In one instance of these GI modifying therapies, ondansetron (Zofran®) is administered 30 minutes to two hours before the start of lonafarnib therapy at 8 mg once daily, up to 8 mg two times daily, up to 8 mg three times daily. In this instance, administration is continued at least for the duration of lonafarnib treatment. In another instance of these GI modifying therapies, granisetron (oral Kytril®) is administered at 2 mg given up to one hour before the start of lonafarnib therapy or 1 mg twice daily. In this instance, administration is continued at least for duration of lonafarnib treatment.

### NK-1 Receptor Antagonists

In one instance of these GI modifying therapies, this GI modifying therapy is an NK-1 receptor antagonist. In one instance of these GI modifying therapies, aprepritant (Emend®) is administered in combination with an 5-HT3 receptor antagonist and a corticosteroid as a three day treatment consisting of a 125 mg dose on day one given one hour before start of lonafarnib therapy, followed by an 80 mg dose on days two and three. In another instance of these GI modifying therapies, fosaprepitant (Emend® IV) is administered in combination with an 5-HT3 receptor antagonist and a corticosteroid (dexamethasone) as a single day treatment consisting of one 150 mg dose of fosaprepitant given up to 30 minutes before start of lonafarnib therapy followed by a single 12 mg dose of dexamethasone and a single dose of a 5-HT3 receptor antagonist such as odansetron, up to a single 150 mg dose of fosaprepitant given up to 30 minutes before start of lonafarnib therapy followed by a single 8 mg dose of dexamethasone and a single dose of a 5-HT3 receptor antagonist such as ondansetron on day one, and a single 8 mg dose of dexamethasone on days 2 through 4.

### Proton Pump Inhibitors

In one instance of these GI modifying therapies, this GI modifying therapy is a proton pump inhibitor (PPI). In one instance of these GI modifying therapies, omeprazole (Prilosec®) is administered in combination with an antacid up to four days before the start of lonafarnib therapy at a dose of 20 mg once daily, up to 40 mg once daily for the duration of lonafarnib therapy. In another instance of these GI modifying therapies, omeprazole/sodium bicarbonate (Zegerid®) is administered at least one hour before a meal and before the start of lonafarnib therapy at a dose of 20 mg once daily, up to 40 mg once daily for the duration of lonafarnib therapy. In another instance of these GI modifying therapies, esomeprazole magnesium (Nexium®) is administered at least one hour before lonafarnib treatment at dose of 20 mg once daily, up to 40 mg once daily, up to 40 mg twice daily for the duration of lonafarnib therapy. In another instance of these GI modifying therapies, esomeprazole strontium is administered at least one hour before lonafarnib treatment at dose of 24.65 mg once daily, up to 49.3 mg once daily, up to 49.3 mg twice daily, for the duration of lonafarnib therapy. In another instance of these GI modifying therapies, lansoprazole (Prevacid®) is administered up to two hours before lonafarnib therapy at a dose of 15 mg once daily of lansoprazole, up to 30 mg once daily, up to 60 mg once daily, up to 30 mg two times daily for a duration up to 14 days, up to 30 mg three times daily for the duration of lonafarnib therapy. In another instance of these GI modifying therapies, dexlansoprazole (Dexilant®) is administered up to two hours before lonafarnib therapy at a dose of 30 mg once daily of dexlansoprazole, up to 60 mg once daily for the duration of lonafarnib therapy. In another instance of these GI modifying therapies, pantoprazole sodium (Protonix®) is administered up to seven days before lonafarnib therapy at a dose of 40 mg once daily, up to 40 mg twice daily for the duration of lonafarnib therapy.

ln some instances of this disclosure, the GI modifying therapy includes administration of a proton pump inhibitor (PPI) selected due to its inhibitory effect on CYP3A4. PPI-mediated inhibition can assist in maintaining therapeutically effective lonafarnib serum levels. Such inhibitory PPIs include, without limitation, omeprazole and rabeprazole.

### Anti-Diarrheal Agents

In one instance of these GI modifying therapies, this therapy is an anti-diarrheal. In one instance of these GI modifying therapies, atropine/diphenoxylate (Lomotil®, Lonox®) is administered at a dose of two Lomotil tablets four times daily or 10 ml of Lomotil® liquid four times daily (20 mg per day) until initial control has been achieved, after which the dosage may be reduced to as little as 5 mg (two tablets or 10 ml of liquid) daily. In another instance of these GI modifying therapies, loperamide HCI (Imodium®) is administered at a dose of 4 mg (two capsules) followed by 2 mg (one capsule) after each unformed stool, up to 16 mg (eight capsules). In another instance of these GI modifying therapies, bismuth subsalicylate (Kaopectate®, Pepto-Bismol®) is administered as 2 tablets or 30 mL every 30 minutes to one hour as needed, up to eight doses in 24 hours.

### V. Pharmaceutical Compositions and Unit Dose Forms

The present specification provides pharmaceutical compositions for providing lonafarnib and ritonavir cotherapy. In one approach, lonafarnib and ritonavir co-administration is combined with prophylactic administration of one, two or three GI stabilizing agents, as discussed in Section IV, above. In one approach, lonafarnib and ritonavir co-administration is combined with interferon co-therapy, as discussed in Section VII, below.

Generally, lonafarnib and ritonavir are formulated for oral administration and administered orally. However, present specification provides methods and compositions for the administration of lonafarnib and/or ritonavir to a human for the treatment of HDV infection using one or more other routes, such as administration of IV or subcutaneous (SQ) formulations. As another example, the methods of this disclosure can be practiced using patch technology, particularly patch technology that employ micro-needles, to administer the drug subcutaneously. Non-oral administration may avoid or at least ameliorate GI and other side effects. Other routes suitable for drug delivery, including systemic and localized routes of administration may be used.

In certain instances, lonafarnib and/or ritonavir may be administered orally in solid dosage forms (e.g., capsules, caplets, tablets, and the like). In certain instances, lonafarnib and/or ritonavir may be administered orally as soft gel capsules comprising liquid,). In some instances, an agent is administers as a, liquid dosage form (oral suspensions, syrups, or elixirs) or a combination (e.g., lonafarnib tablet and ritonavir solution). Liquid dosage forms for oral administration may be provided wherein each dosage unit, for example, teaspoonful, tablespoonful, milliliter, and the like contains a predetermined amount of the composition containing lonafarnib and/or ritonavir.

Lonafarnib and ritonavir may be co-administered separately (as separate unit dosage forms) or may be combined in an oral unit dosage form that comprises both lonafarnib and ritonavir. When administered as separate unit forms, typically the lonafarnib and ritonavir doses are administered (e.g., self-administered) at about the same time, e.g., simultaneously or within about 3 minutes of each other, or alternatively, within about 10, 30 or 60 minutes of each other. In some instances ritonavir is administered before lonafarnib is administered.

Lonafarnib has been manufactured as 50 mg, 75 mg, and 100 mg capsules, but it is within the ability of those of skill in the art to prepare dosage forms with different amounts of the active ingredient. In one instance, a pharmaceutical formulation of this disclosure contains lonafarnib formulated for oral administration as a unit dose form that contains 50 mg, 75 mg, or 100 mg. If a salt or a solvate is used, equivalently larger amounts will be required as is readily understood by the skilled artisan.

Ritonavir is commercially available as 100-mg tablets, 100-mg soft gelatin capsules and an 80-mg/mL oral solution but it is within the ability of those of skill in the art to prepare dosage forms with different amounts of the active ingredient. In various instances, the unit dose form useful in the methods of this disclosure contains 50 mg or 10 mg. If a salt or a solvate is used, equivalently larger amounts will be required as is readily understood by the skilled artisan.

In some embodiments of the invention, as specified by the claims, lonafarnib and ritonavir are delivered in the same dosage form (i.e., "co-formulated"). For example, a dosage form may contain an lonafarnib and ritonavir (along with excipients and auxiliary agents). Without limitation, the lonafarnib and ritonavir may be provided as an admixture, multiparticulate formulation (which may comprise small particles of lonafarnib in a matrix comprising ritonavir, bilayer formulation, a table-within-table formulation, and the like. Such forms for co-administration are well known (see, e.g., US 20090142393, US 20080021078, WO2009042960). Liquid formulations containing both lonafarnib and ritonavir may also be used for coadministration.

Lonafarnib and/or ritonavir dosage forms may be formulated for defined release profiles including immediate release and controlled release (e.g., delayed release or sustained release). For example, lonafarnib may be formulated for delayed release and ritonavir may be formulated for immediate release (whether administered as separate or as a combination dosage form(s).

As described above in Section IV, in one approach it is contemplated that patients receiving lonafarnib will receive prophylactic administration of one or a combination of GI modifying agent(s). In one approach, one or more GI modifying agents is provided as a co-formulation with lonafarnib and/or ritonavir. For example, without limitation lonafarnib, ritonavir and a GI modifying agent may be formulated as a trilayer tablet. In another approach, one or more GI modifying agents provided in a common pharmaceutical package ("co-packaged"), as described below in Section VI. In one approach the one or more GI modifying agents are provided as an immediate release formulation and lonafarnib (and optionally ritonavir) is provided as a controlled release formulation. The agents formulated with different release may be co-packaged and/or co-formulated in a variety of combinations, provided that at least one GI modifying agent is formulated for rapid release and lonafarnib is formulated for controlled (e.g., delayed) release. In a preferred instance, the formulations allow the patient to self-administer lonafarnib and at least one GI modifying agent at substantially the same time (e.g., simultaneously or within about 5 minutes of each other) using formulations that allow the GI modifying agent(s) to take effect prior to release of lonafarnib. Using this approach the patient may have the benefits of prerelease of the GI modifying agent without increasing the number of times per day the patient must self-administer a therapeutic agent.

Methods for making controlled or delayed release formulations are well known in the art. For illustration and not limitation, in some cases, the lonafarnib, and optionally ritonavir, is formulated with a release-delaying agent. Lonafarnib in this formulation may have zero or relatively low release of drug during a lag period after administration to the subject; and then achieves a rapid release ("burst") of drug after the lag period ends. The lag period is typically in the range of about 0.25 to 3 hours, more often in the range of about 0.5 to 2 hours. Many methods are known in the art for providing delayed-burst release, such as by diffusion, swelling, osmotic bursting or erosion (e.g., based on the inherent dissolution of the agent and incorporated excipients); see U.S. Pat. Pub. No. 20110313009.

In some cases, for illustration and not limitation, the release-delaying agent is designed to allow release of lonafarnib and/or ritonavir upon exposure to defined conditions within the body. In one instance, the release-delaying agent is an enteric release agent that allows the release of the drug upon exposure to a characteristic aspect of the gastrointestinal tract. In one instance, the enteric release agent is pH-sensitive and is affected by changes in pH encountered within the gastrointestinal tract (pH sensitive release). The enteric material typically remains insoluble at gastric pH, then allows for release of the active ingredient in the higher pH environment of the downstream gastrointestinal tract (e.g., often the duodenum, or sometimes the colon). In another instance, the enteric material comprises enzymatically degradable polymers that are degraded by bacterial enzymes present in the lower gastrointestinal tract, particularly in the colon. Optionally, the unit dosage form is formulated with a pH-sensitive enteric material designed to result in a release within about 0.25-2 hours when at or above a specific pH. In various instances, the specific pH can for example be about 4.5, 5, 5.5, 6, or 6.5. In particular instances, the pH-sensitive material allows release of at least 80% of the drug within 1 hour when exposed to a pH of about 5.5 or higher. In another instance, the pH-sensitive material allows release of at least 80% of the drug within 1 hour when exposed to a pH of about 6 or higher.

Materials used for enteric release formulations, for example as coatings, are well known in the art, for example, those described in U.S. Pat. Pub. No. 20110313009. Combinations of different enteric materials may also be used. Multi-layer coatings using different polymers may also be applied. In some instances, the enteric materials causes a delay of drug release in the range of about 0.25 to about 3 hours, sometimes about 0.5 to about 4 hours.

Those of ordinary skill in the art can adjust the lag period before delayed-burst release from enteric coated multiparticulates by varying the enteric layer coating weight and composition. For example, where time in the stomach is <4 hours and some amount of protection (1-3 hours) is desired after the dosage form leaves the stomach, then an appropriate level of coating that provides up to 4 hours of protection between administration and drug release. can be prepared. To identify the correct coating weight, samples of multiparticulates would be pulled from the fluid bed coater over a range of coating weights and tested via in vitro dissolution to determine the appropriate coating level. Based on these results, the correct coating weight would be selected. An example of an enteric coated multiparticulate can be found in U.S. Pat. No. 6,627,223.

Lonafarnib and/or ritonavir may be mixed with (e.g., blended, intermixed or in continuous phase with) and/or contained within (e.g., encapsulated within or coated with) one or more release-delaying agents. For example, the delayed-burst release formulation can be in the form of one or more capsules containing lonafarnib and/or ritonavir. In other instances, lonafarnib and/or ritonavir can be in multiparticulate form such as granules, microparticles (beads) or nanoparticles, coated with release delaying agents.

Pharmaceutical formulations and unit dose forms suitable for oral administration are particularly useful in the treatment of chronic conditions and therapies in which the patient self-administers the drug. However, as noted above, is some cases (including but not limited to acute infections and life-threatening conditions, particularly those requiring hospitalization) intravenous formulations are desirable, and the present specification provides such formulations as well. The specification provides pharmaceutical formulations in which lonafarnib and/or ritonavir can be formulated into preparations for injection in accordance with this disclosure by dissolving, suspending or emulsifying it in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. Unit dosage forms for injection or intravenous administration may comprise in a composition as a solution in sterile water, normal saline or another pharmaceutically acceptable carrier. Appropriate amounts of the active pharmaceutical ingredient for unit dose forms of lonafarnib and/or ritonavir are provided herein.

### VI. Kits and Packaging

Lonafarnib, ritonavir, and optionally one or more GI modifying agents, used in treating HDV patients, may be delivered in a pharmaceutical package to HDV patients. Such packaging is intended to improve patient convenience and compliance with the treatment plan. Typically the packaging comprises paper (cardboard) or plastic.

In one instance, the delivery package comprises lonafarnib and ritonavir in defined, therapeutically effective doses in combination in a single unit dosage form or as separate unit doses. The dose of each drug (e.g., in mg) and the form of the unit dose (e.g., tablet, capsule, immediate release, delayed release, etc.) can be any in accord with the recommendations herein.

In one approach the package includes doses suitable for multiple days administration, such as a 1 week or one month. In a preferred approach, in multi-day packs the doses (e.g., tablets) for each administration (e.g., once per day for QD administration, twice per day for BID administration, etc.) are separated from doses to be administered on different days or at different times.

In another instance, the package comprises defined, therapeutically effective doses of lonafarnib, ritonavir, or a combination of lonafarnib and ritonavir, and one or more GI modifying agents, which are combined in a single package, but segregated from one another in separate compartments within said package.

### VII. Interferon cotherapy

Current medical practice to treat HBV infection and/or HBV and HDV co-infection sometimes employs either interferon-alpha or interferon-gamma monotherapy (including treatment with interferon-alpha-2b or a pegylated interferon, such as Pegasys, marketed by Roche, or PEG-Intron, marketed by Merck) or combination therapy with interferon-alpha and a nucleoside or nucleotide analogue, such as adefovir (Hepsera®), entecavir (Baraclude®), lamivudine (Epivir-HBV®, Heptovir®, Heptodin®), telbivudine (Tyzeka®), tenofovir (Viread®), and ribavirin (such as Rebetol® or Copegus®). In accordance with the methods of the present disclosure, lonafarnib is used in combination with one of these standard therapies to treat HDV infection (i.e., HBV and HDV co-infection). In one instance, lonafarnib is administered in combination with other agents, (such as interferon alpha and ritonavir) at lonafarnib doses of 100 mg QD. In an instance, the specification discloses a method for treating HDV infection by administration of at least 100 mg lonafarnib QD or BID in combination with an interferon or ritonavir.

Thus, in one approach, the patient receiving lonafarnib (e.g., lonafarnib in combination with a booster such as ritonavir) is also treated with interferon (e.g., interferon-a). In some instances, both lonafarnib and interferon-a are administered to the patient, and the lonafarnib dose is at least about 50 mg BID or at least or about 75 mg BID or QD. In some instances the lonafarnib dose is 100 mg BID. In some instances, the administration of lonafarnib and the interferon-a is concurrent. In some instances, the administration of lonafarnib and the interferon-a is sequential. In some instances, the interferon-a is pegylated interferon (hereinafter, "Pegasys"). Thus, it is contemplated that HDV-infected patients receiving lonafarnib-ritonavir co-therapy may also be treated with an interferon.

Administration of lonafarnib in combination with other agents, such as interferon alpha and ritonavir (Norvir) provides efficacious therapy at lower doses and/or reduced dosing frequency. This, in some cases, the patient is administered with lonafarnib, ritonavir and interferon-a. In some instances, the Pegasys is administered weekly. In some instances, pegylated interferon (Pegasys) is administered at a dose of 180 micrograms per week. In these instances, dosing of lonafarnib and the interferon is continued for at least 30 days, usually at least about 60 or even 90 days or longer, including 6 months to a year or longer. In an approach administration will be continuous for about 30 days, more typically 30 or 60 days, and often as long 6 months, 9 months, and 12 months. In some instances, dosing will be discontinued after virus levels have decreased to undetectable levels for a period of time (such as 1 to 3 months or longer).

### Interferons

Based on the type of receptor through which they signal, human interferons have been classified into three major types. In various instances, an interferon of any of Types I-III is used in combination with lonafarnib to treat HDV infection. All type I IFNs bind to a specific cell surface receptor complex known as the IFN-alpha receptor (IFNAR) that consists of IFNAR1 and IFNAR2 chains. The type I interferons present in humans are IFN-alpha, IFN-beta, IFN-epsilon, and IFN-omega. Type II IFNs bind to IFN-gamma receptor (IFNGR) that consists of IFNGR1 and IFNGR2 chains. The type II interferon in humans is IFN-gamma. The recently classified type III interferon group consists of three IFN-lambda molecules called IFN-lambda1, IFN-lambda2 and IFN-lambda3 (also called IL29, IL28A, and IL28B, respectively). These IFNs signal through a receptor complex consisting of IL10R2 (also called CRF2-4) and IFNLR1 (also called CRF2-12).

Thus, the present specification provides combination therapies in which an interferon-alpha or interferon-lambda are used in combination with lonafarnib. The term "interferon-alpha" or "IFN-α" and "interferon-lambda" or "IFN-λ" as used herein refers to a family of related polypeptides that inhibit viral replication and cellular proliferation and modulate immune response. The term "IFN-α" includes naturally occurring IFN-α; synthetic IFN-α; derivatized IFN-α (e.g., PEGylated IFN-α, glycosylated IFN-α, and the like); and analogs of naturally occurring or synthetic IFN-α. The term "IFN-α" also encompasses consensus IFN-α. Thus, essentially any IFN-α or IFN-λ that has antiviral properties, as described for naturally occurring IFN-α, can be used in the combination therapies of this disclosure.

Suitable interferons for purposes of this disclosure include, but are not limited to pegylated IFN-α-2a, pegylated IFN-α-2b, consensus IFN and IFN-λ.

The term "IFN-α" encompasses derivatives of IFN-α that are derivatized (e.g., are chemically modified relative to the naturally occurring peptide) to alter certain properties such as serum half-life. As such, the term "IFN-α" includes IFN-α derivatized with polyethylene glycol ("PEGylated IFN-α"), and the like. PEGylated IFN-α, and methods for making same, is discussed in, e.g., U.S. Pat. Nos. 5,382,657; 5,951,974; and 5,981,709. PEGylated IFN-α encompasses conjugates of PEG and any of the above-described IFN-α molecules, including, but not limited to, PEG conjugated to interferon alpha-2a (Roferon, Hoffman La-Roche, Nutley, N.J.), interferon alpha-2b (Intron, Schering-Plough, Madison, N.J.), interferon alpha-2c (Berofor Alpha, Boehringer Ingelheim, Ingelheim, Germany); and consensus interferon as defined by determination of a consensus sequence of naturally occurring interferon alphas (Infergen®, InterMune, Inc., Brisbane, CA.).

Thus, in some instances of the combination therapies of this disclosure, the IFN-α has been modified with one or more polyethylene glycol moieties, i.e., pegylated. Two forms of pegylated-interferon, peginterferon alfa-2a (40 kD) (Pegasys, Hoffmann-La Roche) and peginterferon alfa-2b (12 kD) (Peglntron, Merck), are commercially available, which differ in terms of their pharmacokinetic, viral kinetic, tolerability profiles, and hence, dosing.

Peginterferon alfa-2a (Pegasys) consists of interferon alfa-2a (^{∼}20 kD) covalently linked to a 40 kD branched polyethylene glycol (PEG). The PEG moiety is linked at a single site to the interferon alfa moiety via a stable amide bond to lysine. Peginterferon alfa-2a has an approximate molecular weight of 60,000 daltons. The biologic activity of peginterferon-alfa-2a derives from its interferon alfa-2a moiety which impacts both adaptive and innate immune responses against certain viruses. This alpha interferon binds to and activates human type 1 interferon receptors on hepatocytes which activates multiple intracellular signal transduction pathways, culminating in the expression of interferon-stimulated genes that produce an array of antiviral effects, such as blocking viral protein synthesis and inducing viral RNA mutagenesis. Compared with the native interferon alfa-2a, the peginterferon alfa-2a has sustained absorption, delayed clear. Peginterferon alfa-2a is used as a fixed weekly dose. Peginterferon alfa-2a has a relatively constant absorption after injection and is distributed mostly in the blood and organs.

Peginterferon alfa-2b (Peglntron) consists of interferon alfa-2b covalently linked to a 12 kD linear polyethylene glycol (PEG). The average molecular weight of the molecule is approximately 31,300 daltons. Peginterferon alfa-2b is predominantly composed of monopegylated species (one PEG molecule is attached to one interferon molecule), with only a small amount of dipegylated species. Fourteen different PEG attachment sites on the interferon molecule have been identified. The biologic activity of peginterferon alfa-2b derives from its interferon alfa-2b moiety, which impacts both adaptive and innate immune responses against certain viruses. This alpha interferon binds to and activates human type 1 interferon receptors on hepatocytes which activates multiple intracellular signal transduction pathways, culminating in the expression of interferon-stimulated genes that produce an array of antiviral effects, such as blocking viral protein synthesis and inducing viral RNA mutagenesis. Compared with the native interferon alfa-2b, the peginterferon alfa-2b has sustained absorption, delayed clearance, and a prolonged half-life. Peginterferon alfa-2b is used as a weekly dose based on the weight of the patient. Peginterferon alfa-2b has a rapid absorption and a wider distribution in the body.

The PEG molecule of a PEGylated IFN-α polypeptide is conjugated to one or more amino acid side chains of the IFN-α polypeptide. In one instance, the PEGylated IFN-α contains a PEG moiety on only one amino acid. In another instance, the PEGylated IFN-α contains a PEG moiety on two or more amino acids, e.g., the IFN-α contains a PEG moiety attached to two, three, four, five, six, seven, eight, nine, ten, eleven, twelve, thirteen, or fourteen different amino acid residues. IFN-α may be coupled directly to PEG (i.e., without a linking group) through an amino group, a sulfhydryl group, a hydroxyl group, or a carboxyl group.

Pegylated interferon has been used in the management of HDV as a monotherapy, albeit with clearance of HDV in no more than a quarter of those treated for one year. A combination therapy provided by this disclosure comprises administering lonafarnib as provided herein as a direct antiviral agent with an immune modulator such as interferon (optionally in combination with other antiviral medications). Illustrative interferons include those discussed above. In one instance of these combination therapies, pegylated interferon alfa-2a (Pegasys) is administered weekly in dosages of 180 microgram (mcg) or 135 mcg (used for patients that react negatively to the higher dose) subcutaneously (SQ). In another instance of these combination therapies, pegylated interferon alfa-2b (Peglntron) is administered weekly in dosages of 1.5 mcg/kg/wk SQ. In other instances of these methods, alfa-interferons are used as follows: consensus interferon (Infergen) administered at 9 mcg to 15 mcg SQ daily or thrice weekly; interferon-alfa 2a recombinant administered at 3 MIU to 9 MIU SQ administered thrice weekly; interferon-alfa 2b (Intron A) recombinant administered 3 MIU to 25 MIU SQ administered thrice weekly; and pegylated interferon lambda (IL-28) administered at 80 mcg to 240 mcg SQ weekly.

The term "IFN-α" also encompasses consensus IFN-α. Consensus IFN-α (also referred to as "CIFN" and "IFN-con" and "consensus interferon") encompasses, but is not limited to, the amino acid sequences designated IFN-con1, IFN-con2 and IFN-con3 which are disclosed in U.S. Pat. Nos. 4,695,623 and 4,897,471; and consensus interferon as defined by determination of a consensus sequence of naturally occurring interferon alphas (e.g., Infergen®, Three Rivers Pharmaceuticals, Warrendale, PA). IFN-con1 is the consensus interferon agent in the Infergen® alfacon-1 product. The Infergen® consensus interferon product is referred to herein by its brand name (Infergen®) or by its generic name (interferon alfacon-1). DNA sequences encoding IFN-con may be synthesized as described in the aforementioned patents or other standard methods. In an instance, at least one additional therapeutic agent is CIFN.

The term IFN-λ" encompasses IFN-lambda1, IFN -lambda2, and IFN-lambda3. These proteins are also known as interleukin-29 (IL-29), IL-28A, and IL-28B, respectively. Collectively, these 3 cytokines comprise the type III subset of IFNs. They are distinct from both type I and type II IFNs for a number of reasons, including the fact that they signal through a heterodimeric receptor complex that is different from the receptors used by type I or type II IFNs. Although type I IFNs (IFN-alpha/beta) and type III IFNs (IFN-lambda) signal via distinct receptor complexes, they activate the same intracellular signaling pathway and many of the same biological activities, including antiviral activity, in a wide variety of target cells. Interferon lambda may be administered at any therapeutically appropriate dose, including, without limitation, 80, 120 or 180 mcg QW.

In various instances of the combination therapies of this disclosure, fusion polypeptides comprising an IFN-α and a heterologous polypeptide are used. Suitable IFN-α fusion polypeptides include, but are not limited to, Albuferon-alpha™ (a fusion product of human albumin and IFN-α; Human Genome Sciences; see, e.g., Osborn et al., 2002, J. Pharmacol. Exp. Therap. 303:540-548). Also suitable for use in the present methods are gene-shuffled forms of IFN-α. See, e.g., Masci et al., 2003, Curr. Oncol. Rep. 5:108-113. Other suitable interferons include Multiferon (Viragen), Medusa Interferon (Flamel Technology), Locteron (Octopus), and Omega Interferon (Intarcia/Boehringer Ingelheim).

Thus, in various instances, lonafarnib is dosed in combination with an interferon to treat HDV infection in accordance with the invention. In various instances, the interferon is pegylated IFN-alfa 2a or pegylated IFN-alfa 2b. Suitable doses of lonafarnib/pegylated IFN-alfa 2a are 100 mg BID/180 mcg QW. Suitable doses of lonafarnib/pegylated IFN-alfa 2b are 100 mg BID/1.5 mcg/kg patient weight QW.

### VIII. Other Antiviral Therapies

It is contemplated that HDV-infected patients receiving lonafarnib-ritonavir co-therapy may also be treated with other antiviral agents such nucleosides and nucleotide analogues, compounds used to treat HBV infections, and other agents.

### Nucleosides and Nucleotide Analogs

Antiviral nucleoside or nucleotide analogues that may be used in combination with the lonafarnib-ritonavir cotherapy described herein include such as adefovir (Hepsera®), entecavir (Baraclude®), lamivudine (Epivir-HBV®, Heptovir®, Heptodin®), telbivudine (Tyzeka®), tenofovir (Viread®), and ribavirin (such as Rebetol® or Copegus®).

### Compounds Used to Treat HBV

In various combination therapies of this disclosure, for treatment of HDV, lonafarnib is combined with an antiviral medication directed against HBV. Anti-HBV medications that are currently approved, with the exception of interferons, inhibit reverse transcriptase and are nucleoside or nucleotide analogues. These medications, while effective against HBV, are not effective against HDV as they do not lower HBsAg, which HDV needs to replicate; however, when used in the combination therapies of this disclosure, improved patient outcomes can be achieved. Currently approved anti-HBV medications include: interferon alpha (Intron A®), pegylated interferon (Pegasys®), lamivudine (Epivir-HBV®, Zeffix®, or Heptodin®), adefovir dipivoxil (Hepsera®), entecavir (Baraclude®), telbivudine (Tyzeka®, Sebivo®), clevudine (Korea/Asia), tenofovir (Viread®). Truvada®, which is a combination of tenofovir and emtricitabine, is not yet approved but has been shown to be effective in reducing HBV viral titers in early clinical trials and is useful in the combination therapies of this disclosure.

### Other Therapeutic Compounds

Other therapeutic compounds that may be administered with beneficial effect to an HDV-infected patient that is being treated in accordance with this disclosure include a nucleoside or nucleotide analog; a thiazolide; a protease inhibitor; a polymerase inhibitor; a helicase inhibitor; a Class C CpG toll-like receptor 7 and/or 9 antagonist; an amphipathic helix disruptor or NS4B inhibitor; a statin or other HMG CoA reductase inhibitor; an immunomodulator; an anti-inflammatory; a second prenylation inhibitor; a cyclophilin inhibitor; and an alpha-glucosidase inhibitor.

### Other Therapeutic Modules

Oral lonafarnib-ritonavir cotherapy may be one module in a course of therapies for rapid and complete clearance of HDV infection. Thus, the treatment regimens described herein may be preceded by or followed by complementary therapies.

As one example, some patients may benefit from an initial IV infusion of lonifarnib to achieve high blood levels of the drug rapidly, which levels may be sustained by continuous or periodic IV infusion(s) for some period (one to a few days or perhaps a week) before a patient is placed on the oral therapies more specifically described herein. In these embodiments, a patient may be infused with the drug to achieve the therapeutic efficacy associated with serum levels achieved with 200 mg BID administration (and higher doses). The IV administration is done under a under the care of a physician other trained medical professional (for example, in a hospital), with the prophylactic therapy and monitoring to avoid or ameliorate the AEs associated with oral dosing at those high doses or to discontinue IV administration, if necessary. In other instances, subcutaneous infusions to provide a depot form of the drug that maintains therapeutically effective blood levels of the drug for some days or weeks may be used to achieve the same therapeutically effective results as those described herein for oral therapy.

### IX. Use of Cobicistat as Boosting Agent

While ritonavir is the most widely used CYP3A4 inhibitor, the discoveries described herein allow practice of the lonafarnib combination treatment with CYP3A4 inhibitor inhibitors. In an alternative, the disclosure provides instances, as described elsewhere herein, in which the pharmacokinetic boosting agent Cobicistat is used in combination with lonafarnib in place of ritonavir.

Cobicistat (marketed under the tradename Tybost® by Gilead Sciences) is another potent inhibitor of CYP3A. As does ritonavir, it "boosts" blood levels of other substrates of this enzyme but, unlike ritonavir, it has no anti-viral activity. In addition, while it has a pronounced effect on the enzyme system (CYP3A) responsible for breaking down certain drugs, it does not affect other enzyme systems used by many other medications which may contribute to numerous potentially harmful drug interactions. Cobicistat is useful in the combination therapies of the disclosure at its approved or any lower dose in combination with lonafarnib at any dose and dosing frequency described herein.

In one instance, the disclosure contemplates treatment of HDV patients using the methods and compositions described herein, except that a boosting agent other than ritonavir is used. In one instance the boosting agent is cobicistat. In some instances, a lower dose of cobicistat is used (e.g., 50 mg QD or 50 mg BID).

**TABLE 4** illustrates, without limitation, four exemplary dosing schedules (A-D) for lonafarnib-cobicistat co-therapy. Cobicistat is useful in the combination therapies of the disclosure at its approved or any lower dose in combination with lonafarnib at any dose and dosing frequency described herein. In alternative instances cobicistat is administered at a lower (e.g., 75 mg) and/or more frequent (e.g., BID) dose.

**TABLE 4**

| | A | B | C | D |
|---|---|---|---|---|
| Dose lonafarnib | 75 mg BID | 50 mg BID | 50 mg QD | 75 mg QD |
| Dose cobicistat | 150 mg QD | 150 mg QD | 150 mg QD | 150 mg QD |

In another instance cobicistat (Tybost®) is administered at 150 mg once daily. In an instance, lonafarnib may be dosed at 100 mg QD, 100 mg BID, optionally in combination with interferon as described above.

### X. Examples

### Example 1. Treating HDV Patients with 100 mg lonafarnib administered BID

### (useful for understanding the invention)

This example demonstrates lonafarnib's efficacy to reduce HDV RNA levels in patients with chronic HDV. The eight Group 1 patients (all with chronic HDV) were treated as follows: Six patients (patients 1, 2, 4, 5, 6, and 8) with chronic delta hepatitis (HDV) were treated with lonafarnib and 2 patients (patients 3 and 7) were administered placebo for a duration of twenty-eight days. The 6 patients in the active treatment group were dosed at 100 mg BID (orally administered) for 28 days. The mean change in HDV RNA levels from baseline to nadir in the lonafarnib active treatment group was -0.74 log HDV RNA copies/mL and in the placebo group was -0.24 log HDV RNA copies/mL.

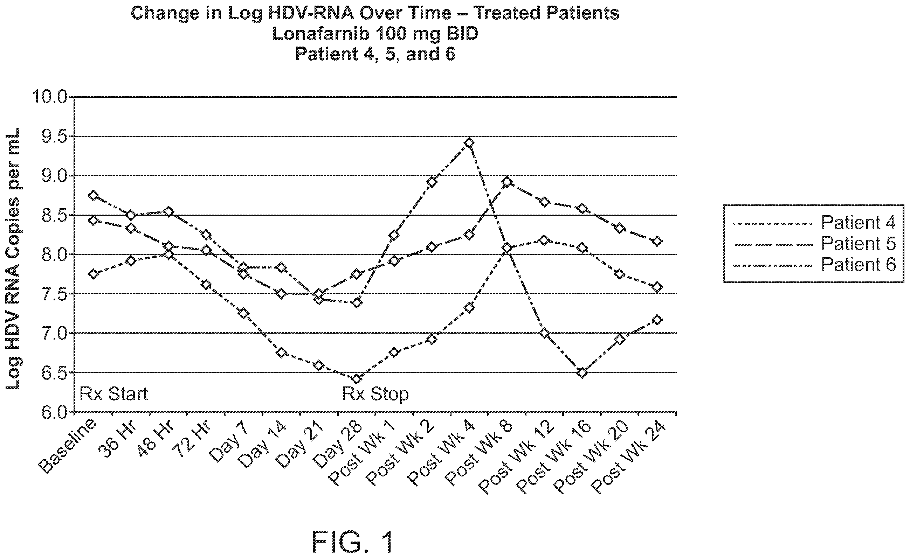

Patients 4, 5, 6 and 8 were responsive to therapy, as defined by greater than or equal to a 0.5 log HDV RNA copies/mL decline in quantitative serum HDV RNA levels from baseline to nadir during active treatment. See TABLE 5 (showing change in HDV RNA viral load for each patient, during treatment and post-treatment) and FIGURE 1 (showing the time course of the log HDV RNA copies/mL levels of Patients 4, 5, and 6). The change in Patient 4's HDV RNA levels from baseline to the end of the treatment (EOT) was -1.34 log HDV RNA copies/mL. The change in Patient 5's HDV RNA levels from baseline to EOT was - 0.82 log HDV RNA copies/mL. The change in Patient 6's HDV RNA levels from baseline to EOT was -1.41 log HDV RNA copies/mL.

**TABLE 5**

| 100 mg BID Lonafarnib Monotherapy Baseline vs EOT on Rx vs Post | | | | | | | | |
|---|---|---|---|---|---|---|---|---|
| | Patient 1 | Patient 2 | Patient 4 | Patient 5 | Patient 6 | Patient 8 | Patient 3 | Patient 7 |
| Dose lonafarnib (mg BID) | 100 | 100 | 100 | 100 | 100 | 100 | Placebo | Placebo |
| Composite Baseline (log copies/mL) | 7.33 | 8.08 | 7.77 | 8.59 | 8.78 | 7.19 | 7.29 | 8.33 |
| Change log Baseline to EOT | -0.20 | -0.19 | -1.34 | -0.82 | -1.41 | -0.46 | -0.29 | -0.14 |
| Change log Baseline to post-FU | -0.10 | +0.27 | -0.16 | -0.43 | -1.63 | +0.20 | -0.10 | -0.13 |

### HDV RNA viral load correlates with plasma concentrations of lonafarnib.

**FIGURE 2** illustrates the correlation between plasma levels of lonafarnib and viral load. Patients with higher plasma levels of lonafarnib experienced greater declines in HDV RNA titers during treatment. Plasma levels ranged between 200 ng/mL and 1,100 ng/mL during treatment.

### Post-treatment viral rebound

Patients 4, 5, and 6 exhibited viral rebound or an increase in serum HDV RNA levels after lonafarnib therapy was discontinued on day 28. Patient 4's HDV RNA level increased 1.7 log HDV RNA copies/mL after discontinuation of lonafarnib. Patient 5's HDV RNA level increased 1.4 log HDV RNA copies/mL after discontinuation of lonafarnib. Patient 6's HDV RNA level increased after discontinuation of lonafarnib. Patients 4, 5, and 6 exhibited subsequent decreases in HDV RNA levels, beginning approximately 4-8 weeks after lonafarnib therapy was discontinued, which is considered to be attributed to virus to virus dynamics between HDV RNA and HBV DNA.

### Example 2. Treating HDV Patients with 200 mg and 300 mg lonafarnib administered BID

### (useful for understanding the invention)

Six human subjects known to be infected with HDV, as documented by baseline HDV RNA viral titers ranging from 5.8 log HDV RNA copies/mL to 8.78 log HDV RNA copies/mL, were treated with lonafarnib at doses of either 200 mg BID or 300 mg BID for a period of 84 days.

### Effect of 28 Days Treatment

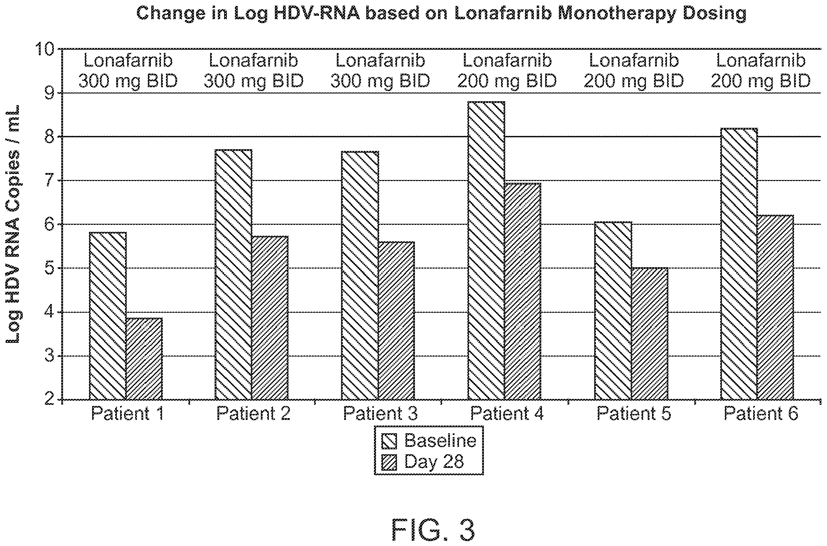

At the end of 28 days of treatment, the mean change in viral load across the six subjects from baseline to day 28 was -1.63 log copies/mL for the 200 mg BID group and - 2.00 log copies/mL for the 300 mg BID group. See **TABLE 6** and **FIGURE 3****.**

**TABLE 6**

| | 300 mg BID lonafarnib | | | 200 mg BID lonafarnib | | |
|---|---|---|---|---|---|---|
| | Patient 1 | Patient 2 | Patient 3 | Patient 4 | Patient 5 | Patient 6 |
| HDV RNA viral load baseline (log copies/mL) | 5.8 | 7.7 | 7.66 | 8.78 | 6.06 | 8.19 |
| Δ log Baseline to Day 28 | -1.95 | -1.97 | -2.07 | -1.85 | -1.06 | -1.98 |

The results at Day 28 demonstrated superior efficacy of the 200 mg BID and 300 mg BID administration schedule over the 100 mg BID administration schedule. However, additional efficacy is required for significant therapeutic benefit. Based on the results of treating HDV patients with 200 mg and 300 mg lonafarnib administered BID, we determined that strategies for achieving significant therapeutic benefit include continuing the administration for at least an additional 30 days or an additional 60 days or longer in combination with a boosting agent. In some instances, the use of a boosting agent, alone or in combination with an interferon, can enable patients to achieve significant therapeutic benefit with a lower lonafarnib dose (e.g., 100 mg QD or 100 mg BID) or shorter duration of treatment (e.g., 30 days).

### Effect of 56-84 Day Treatment

When 200 mg BID and 300 mg BID dosing was continued for 56 days (days 29-56 may be referred to as Month 2) and 84 days (days 57-84 may be referred to as Month 3), the change in viral loads across the six patients either plateaued or increased from viral load levels at 28 days. This no change or rise in viral loads was attributed to poor gastrointestinal tolerance to lonafarnib. These six patients did not receive GI modifying agents prophylactically to mitigate gastrointestinal distress. It is likely that compliance was poor to 200 mg BID and 300 mg BID lonafarnib treatment in month 2 and month 3 (from days 29-84) due to GI intolerance.

### Example 3. Combination Treatment of HDV Patients with 100 mg BID lonafarnib and interferon

### (useful for understanding the invention)

Three human subjects known to be infected with HDV, as documented by baseline HDV RNA viral titers ranging from 4.34 log HDV RNA copies/mL to 5.15 log HDV RNA copies/mL and ALT values ranging from 155-174 IU/L, were treated with lonafarnib in doses of 100 mg BID in combination with Pegasys (peg interferon alfa-2a) 180 µg per week for a period of 56 days (2 month).

At the end of 28 days, all three patients' HDV RNA viral titers had decreased from baseline, ranging from -1.04 log HDV RNA copies/mL to -2.00 log HDV RNA copies/mL drop in HDV-RNA, with an average drop across the three subjects of -1.8 log HDV RNA copies/mL. At the end of 56 days, all three patients' HDV RNA viral titers continued to decline with a mean viral load decline of 3 log copies/mL at day 56. In addition, the ALT values of all three patients decreased from baseline through day 56, continuing to decline after therapy was stopped, normalizing in two of three patients by post week 4 of treatment cessation. Upper limit of normal for ALT values is estimated to be 40 U/L.

The change in HDV RNA viral load and ALT values for each patient is tabulated below in **TABLE 7.**

**TABLE 7**

| Lonafarnib 100 mg BID + PEG IFN 180 mcg QW | | | | | | | | |
|---|---|---|---|---|---|---|---|---|
| | Log HDV RNA copies/mL | | | | | | | ALT (U/L)* |
| Patient | Day 1 | Day 2 | Day 3 | Day 7 | Day 14 | Day 28 | Change | Day 1 to Day 28 to Day 56 to Post Wk 4** |
| 1 | 5.15 | 5.28 | 4.98 | 4.32 | 4.04 | 3.64 | -1.51 | 161→73→66→28 |
| 2 | 5.04 | 5.15 | 5.04 | 4.43 | 4.34 | 4.00 | -1.04 | 174→144→69→55 |
| 3 | 4.34 | 4.15 | 4.04 | 4.04 | 2.34 | 2.34 | -2.00 | 155→80→56→40 |

| | | | | | | | | |
|---|---|---|---|---|---|---|---|---|
| *Upper limit of normal = 40 U/L **Post Wk 4 refers to the end of the first week following termination of treatment. | | | | | | | | |

These viral load results demonstrate comparable efficacy to the 200 mg BID lonafarnib regimen and superior efficacy over the 100 mg BID lonafarnib regimen and fewer grade 2 adverse events (AEs) compared to 200 mg BID lonafarnib.

### Example 4. Combination Treatment of HDV Patients with 100 mg BID lonafarnib and 100 mg

### QD Ritonavir

Three human subjects known to be infected with HDV, as documented by baseline HDV RNA viral titers ranging from 5.14 log HDV RNA copies/mL to 6.83 log HDV RNA copies/mL and ALT values of 84-195 U/L, were treated with lonafarnib at doses of 100 mg BID in combination with ritonavir at doses of 100 mg QD for a period of 8 weeks, substantially as otherwise described in Example 1.

At the end week 4, all three patients' HDV RNA viral titers had decreased from baseline, ranging from -1.71 log HDV RNA copies/mL to at least -2.76 log HDV RNA copies/mL drop in HDV-RNA, with an average drop across the three patients of -2.2 log HDV RNA copies/mL. At the end of week 8, all three patients' HDV RNA viral titers continued to decrease, with patient 2's viral titers being undetectable. The average viral load decline across all three patients at week 8 was -3.2 log HDV-RNA.

In addition, all three subjects' ALT values decreased from baseline, ranging from 35-50 U/L. Upper limit of normal for ALT values is estimated to be 40 U/L. See **TABLE 8.**

The change in HDV RNA viral load for each patient is tabulated below in See **TABLE** 8 and shown in **FIGURE 5**.

**TABLE 8**

| **100 mg lonafarnib BID + 100 mg ritonavir QD** | | | | | | | | | |
|---|---|---|---|---|---|---|---|---|---|
| | **Log HDV RNA copies/mL** | | | | | | | | **ALT (U/L)*** |
| **Patient** | Baseline | Wk 1 | Wk 2 | Wk 4 | Wk 5 | Wk 6 | Wk 8 | Change | Baseline to Wk 4 to Wk 8 |
| **1** | 6.34 | 4.96 | 4.40 | 4.63 | 4.48 | 4.18 | 3.97 | -2.37 | 83→50→43 |
| **2** | 5.14 | 4.21 | 3.30 | 2.38 | 1.86 | 2.08 | ND** | > -5 | 206→58→32 |
| **3** | 6.83 | 5.82 | 5.38 | 4.68 | 4.62 | 4.23 | 3.99 | -2.84 | 72→35→43 |

| | | | | | | | | | |
|---|---|---|---|---|---|---|---|---|---|
| * Upper limit of normal = 40 U/L **Below detection limit. | | | | | | | | | |

These results demonstrate superior efficacy compared to 200 mg BID and 300 mg BID lonafarnib monotherapy and over the combination treatment of 100 mg BID lonafarnib and Pegasys (peg interferon alfa-2a) 180 µg per week. In addition, there were fewer grade 2 AEs observed as compared to 200 mg BID lonafarnib. See **TABLE 9.**

**TABLE 9**

| | N=3 | | | | N=3 | | | | N=3 | | | | N=3 | | | |
|---|---|---|---|---|---|---|---|---|---|---|---|---|---|---|---|---|
| | LNF 200 mg BID | | | | LNF 300 mg BID | | | | LNF 100 mg BID | | | | LNF 100 mg BID | | | |
| | | | | | | | | | RTN 100 mg QD | | | | PEG IFN 180 mcg QW | | | |
| Grade | 1 | 2 | 3 | 4 | 1 | 2 | 3 | 4 | 1 | 2 | 3 | 4 | 1 | 2 | 3 | 4 |
| Nausea | | ✔ | | | | ✔ | | | ✔ | | | | | ✔ | | |
| Diarrhea | | ✔ | | | | ✔ | | | | ✔ | | | ✔ | | | |
| Fatique | | ✔ | | | | ✔ | | | | ✔ | | | | ✔ | | |
| Wt Loss | | ✔ | | | | ✔ | | | ✔ | | | | ✔ | | | |
| Anorexia | | ✔ | | | | ✔ | | | | ✔ | | | | ✔ | | |

**TABLE 10**

| Mean Serum Concentrations (ng/mL) | | | | |
|---|---|---|---|---|
| | **N=3** | **N=3** | **N=3** | **N=3** |
| | **LNF 200 mg BID** | **LNF 300 mg BID** | **LNF 100 mg BID** | **LNF 100 mg BID** |
| | | | **RTN 100 mg QD** | **Peg IFN 180 mcg QW** |
| Day 7 | 2555 | 2994 | 2745 | 587 |
| Day 14 | 2959 | 1548 | 2720 | 896 |
| Day 21 | | | | |
| Day 28 | 2771 | 1984 | 2741 | 572 |
| Day 35 | | | 2860 | 1325 |
| Day 42 | | | 3633 | |
| Day 56 | 1656 | 2032 | 3767 | 643 |
| Day 84 | 443 | 977 | | |

### Example 5. Treatment of HDV Patients with Lonafarnib and Ritonavir

The example describes the anti-HDV effect of the combination therapy of lonafarnib and ritonavir. Eight patients with chronic HDV infection were treated with four different dose combinations of lonafarnib and ritonavir (orally administered) for 84 days under the regimens summarized in **TABLE 10.**

### Results

The changes in patients' HDV RNA levels from baseline as a result of lonafarnib and ritonavir combination therapy are summarized in **TABLE 11.**

**TABLE 11**

| Lonafarnib-Ritonavir Co-therapy | | | | | | | | |
|---|---|---|---|---|---|---|---|---|
| | Patient 1 | Patient 2 | Patient 3 | Patient 4 | Patient 5 | Patient 6 | Patient 7 | Patient 8 |
| Group | Group 1 | | Group 2 | | Group 3 | Group 4 | | |
| Dose lonafarnib | 100 mg BID | | 100 mg BID | | 100 mg QD | 150 mg QD | | |
| Dose ritonavir | 100 mg QD | | 50 mg BID | | 100 mg QD | 100 mg QD | | |
| Change log VL Baseline to Nadir | -2.32 | -3.37 | -2.37 | -1.99 | -2.05 | -2.12 | -1.45 | -1.39 |
| Change log VL Baseline to Day 28 | -2.32 | -2.45 | -2.37 | -1.94 | na | -2.01 | -1.39 | -1.39 |
| Change log VL Baseline to Day 56 | -1.56 | -2.23 | -1.79 | -1.20 | -1.58 | -1.61 | -1.00 | -0.70 |
| Change log VL Baseline to Day 84* | -1.47 | -0.61 | 1.09 | -0.62 | -2.05 | -2.00 | -0.71 | 0.21 |
| LNF Serum Concentration at Week 4 (ng/mL) | 3101 | 4827 | 3474 | 4580 | No data | 1411 | 1198 | 2042 |
| LNF Serum Concentration at Week 8 (ng/mL) | 1678 | 1484 | 3070 | 2969 | 1502 | 1662 | 1475 | 3139 |
| LNF Serum Concentration at Week 12 (ng/mL) | 1243 | 0 | 2377 | 423 | 1337 | 1807 | 1727 | 0 |

| | | | | | | | | |
|---|---|---|---|---|---|---|---|---|
| *See **TABLE 11** for discussion of dose reductions in weeks 7-12 | | | | | | | | |

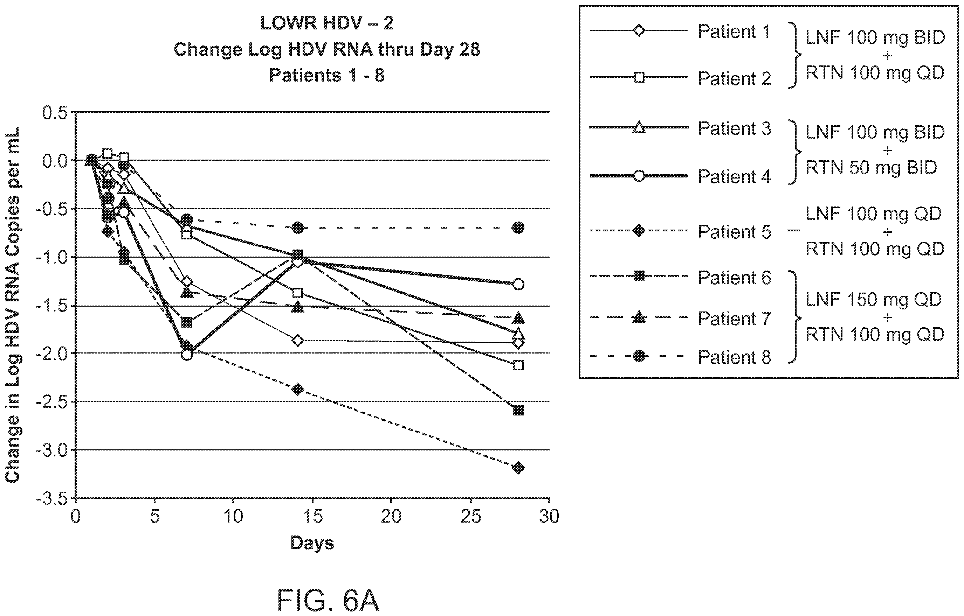

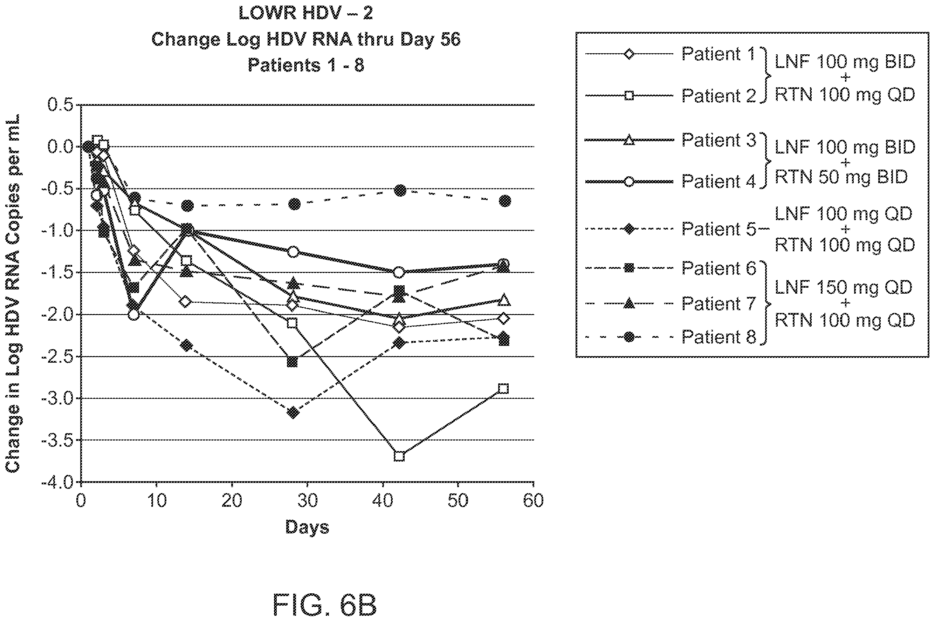

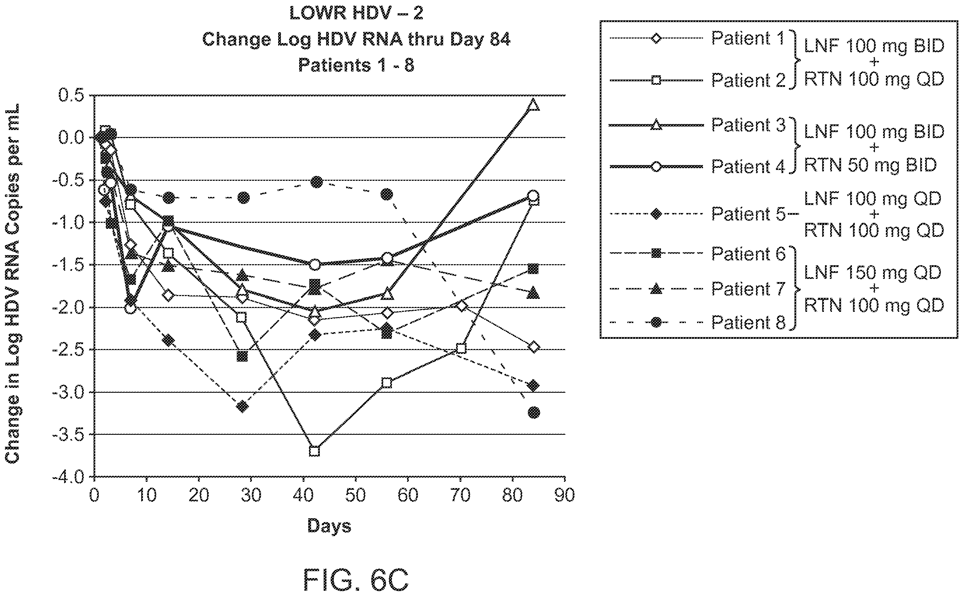

Time courses showing changes in HDV RNA levels in patients 1-8 through Day 28, Day 56 and Day 84 are shown in **FIGURES 6A****.****1****,** **6A****.****2****,** **6A****.****3****,** respectively (change relative to a normalized baseline). The mean change in log viral load is -1.89 after Day 28, -1.86 after Day 56 and -1.62 after Day 84. Nadirs are reached between week 4 to 6, after which VL plateaus or in some cases, elevate slightly. Group 4 may have reached a saturable absorption point, which suggests a lower lonafarnib dose is preferred.

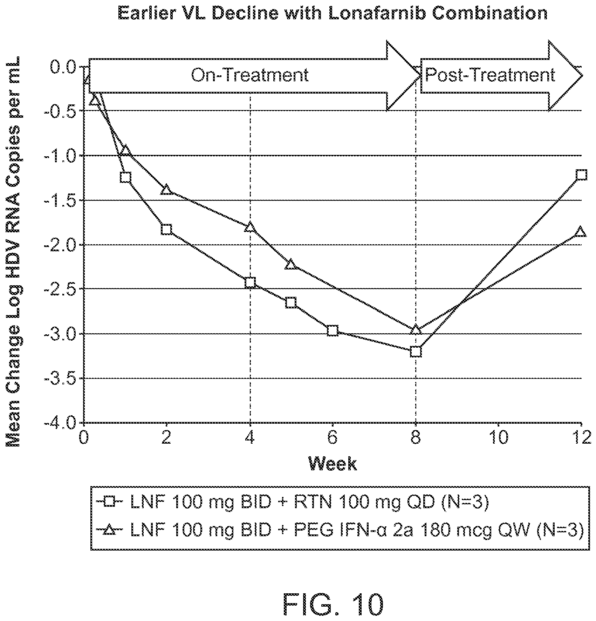

Groups 1 and 2 maintained the highest Cmin. These two groups have either BID lonafarnib (Group 1) or BID ritonavir (Group 2). This suggests higher or more frequent ritonavir doses may be beneficial, such as BID. **FIGURE 10** illustrates that QD dosing of ritonavir (as shown in the graph) provides LNF serum concentrations that are in the 2500-3500 ng/mL range. Increasing ritonavir dosing to BID in patients may achieve higher lonafarnib serum concentrations of >5000 ng/mL.

The correlation of increasing serum levels of lonafarnib with decreasing levels of HDV RNA in hepatitis delta infected patients treated with lonafarnib-ritonavir cotherapy is exemplified in patient 2 (Figure 7) and patient 8 (Figure 8). Patients with serum lonafarnib levels below 2000 ng/mL, in patient 8 for example, are likely to have lower viral load declines (< 1.5 log HDV-RNA) compared to patients with lonafarnib levels approaching or greater than 5,000 ng/mL (> 2 log HDV-RNA).

### Adverse Effects

**TABLE 12** summarizes patients' adverse events during the first 6 weeks of therapy, and shows that 75% of patients in the study (six of eight patients) required at least one dose reduction in weeks 7-10. Dose reductions often resulted in a rise or plateau in HDV RNA levels.

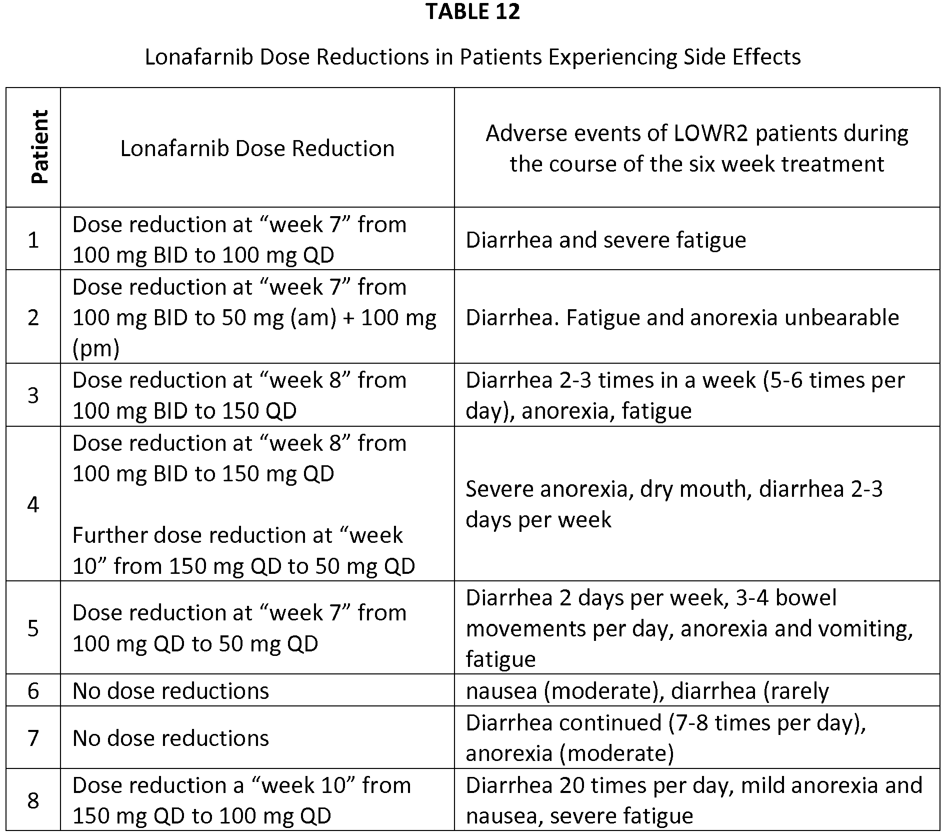

Lonafarnib dose was reduced in 6 of 8 patients (patients 1, 2, 3, 4, 5, 8) due to side effects. Dose reductions correlated with viral load plateau or increase. Lomotil and ondansetron were used by three of the eight patients (beginning at Week 4). Of the three, two did not require lonafarnib dose reduction.

### Example 6

This prophetic example describes treating HDV infection by administration of lonafarnib 50 mg QD + Ritonavir 100 mg QD. A patient infected with HDV self-administers the following regimen daily for 90-180 days:
lonafarnib 50 mg QD
Ritonavir 100 mg QD

During the course of the treatment the patient's lonafarnib serum levels and HDV viral load are determined periodically. After 90 days of treatment the patient's viral load is reduced over baseline.

### Example 7

This prophetic example describes treating HDV infection by administration of lonafarnib 50 mg BID + Ritonavir 50 mg BID. A patient infected with HDV self-administers the following regimen daily for 90-180 days:
lonafarnib 50 mg BID
Ritonavir 50 mg BID
ondansetron 8 mg BID
lomotil 5 mg BID
famotidine 20 mg BID.

During the course of the treatment the patient's lonafarnib serum levels and HDV viral load are determined periodically. After 90 days of treatment the patient's viral load is reduced over baseline.

### Example 8

This prophetic example describes treating HDV infection by administration of lonafarnib 75 mg QD + Ritonavir 100 mg QD. A patient infected with HDV self-administers the following regimen daily for 90-180 days:
lonafarnib 75 mg QD
Ritonavir 100 mg QD
ondansetron 8 mg BID
lomotil 5 mg BID
famotidine 20 mg BID.

During the course of the treatment the patient's lonafarnib serum levels and HDV viral load are determined periodically. After 90 days of treatment the patient's viral load is reduced over baseline.

### Example 9

This prophetic example describes treating HDV infection by administration of lonafarnib 75 mg BID + Ritonavir 50 BID. A patient infected with HDV self-administers the following regimen daily for 90-180 days:
lonafarnib 75 mg BID
Ritonavir 50 mg BID
ondansetron 8 mg BID
lomotil 5 mg BID*
famotidine 20 mg BID
omeprazole 20 mg BID
*titrate to dose

During the course of the treatment the patient's lonafarnib serum levels and HDV viral load are determined periodically. After 90 days of treatment the patient's viral load is reduced over baseline.

Each of the narrower species and subgeneric groupings falling within the generic disclosure also form part of the disclosure. In addition, where features or aspects of the disclosure are described in terms of Markush groups, those skilled in the art will recognize that the disclosure is also thereby described in terms of any individual member or subgroup of members of the Markush group.

## Claims

1. Lonafarnib and ritonavir for use in the treatment of hepatitis delta virus (HDV) infection in a human patient, wherein lonafarnib and ritonavir are administered orally to the patient as a co-therapy.

2. Lonafarnib and ritonavir for use according to claim 1, wherein lonafarnib is administered orally at a total daily dose in the range of 50 mg to 150 mg and ritonavir is administered orally at a total daily dose in the range of 100 mg to 200 mg.

3. Lonafarnib and ritonavir for use according to claim 2, wherein lonafarnib is administered orally at a dose of 50 mg BID and ritonavir is administered orally at a dose of 100 mg BID.

4. Lonafarnib and ritonavir for use according to any of claims 1 to 3, wherein lonafamib and ritonavir are administered to the patient as a co-therapy for at least 90 days.

5. Lonafarnib and ritonavir for use according to any of claims 1 to 4, wherein the lonafarnib and ritonavir are administered as (i) separate unit dose forms of lonafarnib and ritonavir that are administered at about the same time; or (ii) a single unit dose form.

6. Lonafarnib and ritonavir for use according to any of claims 1 to 5, wherein the patient is also administered interferon, preferably wherein the interferon is interferon alpha or interferon lambda, more preferably wherein the interferon alpha is pegylated interferon alpha-2a.

7. Lonafarnib and ritonavir for use according to claim 6, wherein the interferon is interferon lambda or pegylated interferon alpha-2a and is administered at a dose of 180 µg QW.

8. Lonafarnib and ritonavir for use according to claim 6, wherein the interferon is interferon lambda and is administered at a dose of 120 µg QW.

9. Lonafarnib and ritonavir for use according to any of claims 1 to 8, wherein the patient is also administered one or more gastrointestinal modifying agents.

10. Lonafarnib for use in the treatment, by oral administration, of hepatitis delta virus (HDV) infection in a human patient, wherein the patient is also orally administered ritonavir as a co-therapy.

11. Lonafarnib for use according to claim 10, wherein lonafarnib is administered orally at a total daily dose in the range of 50 mg to 150 mg and ritonavir is administered orally at a total daily dose in the range of 100 mg to 200 mg.

12. Lonafarnib for use according to claim 11, wherein the total daily dose of lonafarnib is 100 mg.

13. Lonafarnib for use according to claim 11 or claim 12, wherein the lonafarnib is administered orally at a dose of 50 mg BID and the ritonavir is administered orally at a dose of 100 mg BID.

14. Lonafarnib for use according to any of claims 10 to 13, wherein lonafamib and ritonavir are administered to the patient as a co-therapy for at least 90 days.

15. Lonafarnib for use according to any of claims 10 to 14, wherein the lonafarnib and ritonavir are administered as (i) separate unit dose forms of lonafarnib and ritonavir that are administered at about the same time; or (ii) a single unit dose form.

16. Lonafarnib for use according to any of claims 10 to 15, wherein the patient is also administered interferon, preferably wherein the interferon is interferon alpha or interferon lambda, more preferably wherein the interferon alpha is pegylated interferon alpha-2a.

17. Lonafarnib for use according to claim 16, wherein the interferon is interferon lambda or pegylated interferon alpha-2a and is administered at a dose of 180 µg QW.

18. Lonafarnib for use according to claim 16, wherein the interferon is interferon lambda and is administered at a dose of 120 µg QW.

## Patentansprüche

1. Lonafarnib und Ritonavir zur Verwendung bei der Behandlung einer Hepatitis-delta-Virus(HDV)-Infektion bei einem humanen Patienten, wobei das Lonafarnib und das Ritonavir dem Patienten als eine Begleittherapie oral verabreicht werden.

2. Lonafarnib und Ritonavir zur Verwendung nach Anspruch 1, wobei das Lonafarnib in einer täglichen Gesamtdosis im Bereich von 50 mg bis 150 mg oral verabreicht wird und das Ritonavir in einer täglichen Gesamtdosis im Bereich von 100 mg bis 200 mg oral verabreicht wird.

3. Lonafarnib und Ritonavir zur Verwendung nach Anspruch 2, wobei das Lonafarnib zweimal täglich in einer Dosis von 50 mg oral verabreicht wird und das Ritonavir zweimal täglich in einer Dosis von 100 mg oral verabreicht wird.

4. Lonafarnib und Ritonavir zur Verwendung nach einem der Ansprüche 1 bis 3, wobei das Lonafarnib und das Ritonavir an den Patienten als eine Begleittherapie für mindestens 90 Tage verabreicht werden.

5. Lonafarnib und Ritonavir zur Verwendung nach einem der Ansprüche 1 bis 4, wobei das Lonafarnib und das Ritonavir verabreicht werden als (i) separate Einheitsdosisformen von Lonafarnib und Ritonavir, die etwa zur selben Zeit verabreicht werden, oder (ii) eine einzelne Einheitsdosisform.

6. Lonafarnib und Ritonavir zur Verwendung nach einem der Ansprüche 1 bis 5, wobei dem Patienten ebenfalls Interferon verabreicht wird, wobei das Interferon vorzugsweise Interferon alpha oder Interferon lambda ist, wobei das Interferon alpha besser noch pegyliertes Interferon alpha-2a ist.

7. Lonafarnib und Ritonavir zur Verwendung nach Anspruch 6, wobei das Interferon Interferon lambda oder pegyliertes Interferon alpha-2a ist und einmal pro Woche in einer Dosis von 180 µg verabreicht wird.

8. Lonafarnib und Ritonavir zur Verwendung nach Anspruch 6, wobei das Interferon Interferon lambda ist und einmal pro Woche in einer Dosis von 120 µg verabreicht wird.

9. Lonafarnib und Ritonavir zur Verwendung nach einem der Ansprüche 1 bis 8, wobei dem Patienten ebenfalls ein oder mehrere verdauungstraktmodifizierende Mittel verabreicht werden.

10. Lonafarnib zur Verwendung bei der Behandlung einer Hepatitis-delta-Virus(HDV)-Infektion bei einem humanen Patienten durch eine orale Verabreichung, wobei dem Patienten ebenfalls Ritonavir als eine Begleittherapie oral verabreicht wird.

11. Lonafarnib zur Verwendung nach Anspruch 10, wobei das Lonafarnib oral in einer täglichen Gesamtdosis im Bereich von 50 mg bis 150 mg verabreicht wird und das Ritonavir oral in einer täglichen Gesamtdosis im Bereich von 100 mg bis 200 mg verabreicht wird.

12. Lonafarnib zur Verwendung nach Anspruch 11, wobei die tägliche Gesamtdosis von Lonafarnib 100 mg beträgt.

13. Lonafarnib zur Verwendung nach Anspruch 11 oder Anspruch 12, wobei das Lonafarnib oral in einer Dosis von zweimal täglich 50 mg verabreicht wird und das Ritonavir oral in einer Dosis von zweimal täglich 100 mg verabreicht wird.

14. Lonafarnib zur Verwendung nach einem der Ansprüche 10 bis 13, wobei das Lonafarnib und das Ritonavir dem Patienten als eine Begleittherapie für mindestens 90 Tage verabreicht werden.

15. Lonafarnib zur Verwendung nach einem der Ansprüche 10 bis 14, wobei das Lonafarnib und das Ritonavir verabreicht werden als (i) separate Einheitsdosisformen von Lonafarnib und Ritonavir, die etwa zur selben Zeit verabreicht werden; oder (ii) eine einzelne Einheitsdosisform.

16. Lonafarnib zur Verwendung nach einem der Ansprüche 10 bis 15, wobei dem Patienten ebenfalls Interferon verabreicht wird, wobei das Interferon vorzugsweise Interferon alpha oder Interferon lambda ist, wobei das Interferon alpha besser noch pegyliertes Interferon alpha-2a ist.

17. Lonafarnib zur Verwendung nach Anspruch 16, wobei das Interferon Interferon lambda oder pegyliertes Interferon alpha-2a ist und einmal pro Woche in einer Dosis von 180 µg verabreicht wird.

18. Lonafarnib zur Verwendung nach Anspruch 16, wobei das Interferon Interferon lambda ist und einmal pro Woche in einer Dosis von 120 µg verabreicht wird.

## Revendications

1. Lonafarnib et ritonavir destinés à être utilisés dans le traitement de l'infection par le virus de l'hépatite delta (VHD) chez un patient humain, dans lesquels le lonafamib et le ritonavir sont administrés par voie orale au patient comme une thérapie combinée.

2. Lonafarnib et ritonavir destinés à être utilisés selon la revendication 1, dans lesquels le lonafarnib est administré par voie orale à une dose quotidienne totale dans la plage de 50 mg à 150 mg et le ritonavir est administré par voie orale à une dose quotidienne totale dans la plage de 100 mg à 200 mg.

3. Lonafarnib et ritonavir destinés à être utilisés selon la revendication 2, dans lesquels le lonafarnib est administré par voie orale à une dose de 50 mg deux fois par jour et le ritonavir est administré par voie orale à une dose de 100 mg deux fois par jour.

4. Lonafarnib et ritonavir destinés à être utilisés selon l'une quelconque des revendications 1 à 3, dans lesquels le lonafarnib et le ritonavir sont administrés au patient comme une thérapie combinée pendant au moins 90 jours.

5. Lonafarnib et ritonavir destinés à être utilisés selon l'une quelconque des revendications 1 à 4, dans lesquels les lonafarnib et ritonavir sont administrés comme (i) des formes galéniques unitaires de lonafarnib et de ritonavir qui sont administrées au même moment environ ; ou (ii) une forme galénique unitaire unique.

6. Lonafarnib et ritonavir destinés à être utilisés selon l'une quelconque des revendications 1 à 5, dans lesquels le patient reçoit aussi l'administration d'interféron, préférablement dans lesquels l'interféron est l'interféron alpha ou l'interféron lambda, plus préférablement dans lesquels l'interféron alpha est l'interféron pégylé alpha-2a.

7. Lonafarnib et ritonavir destinés à être utilisés selon la revendication 6, dans lesquels l'interféron est l'interféron lambda ou l'interféron pégylé alpha-2a et est administré à une dose de 180 µg une fois par semaine.

8. Lonafarnib et ritonavir destinés à être utilisés selon la revendication 6, dans lesquels l'interféron est l'interféron lambda et est administré à une dose de 120 µg une fois par semaine.

9. Lonafarnib et ritonavir destinés à être utilisés selon l'une quelconque des revendications 1 à 8, dans lesquels le patient reçoit aussi l'administration d'un ou de plusieurs agents de modification gastro-intestinaux.

10. Lonafarnib destiné à être utilisé dans le traitement, par voie orale, de l'infection par le virus de l'hépatite delta (VHD) chez un patient humain, dans lequel le patient reçoit aussi l'administration par voie orale de ritonavir comme une thérapie combinée.

11. Lonafarnib destiné à être utilisé selon la revendication 10, dans lequel le lonafarnib est administré par voie orale à une dose quotidienne totale dans la plage de 50 mg à 150 mg et le ritonavir est administré par voie orale à une dose quotidienne totale dans la plage de 100 mg à 200 mg.

12. Lonafarnib destiné à être utilisé selon la revendication 11, dans lequel la dose quotidienne totale de lonafarnib est 100 mg.

13. Lonafarnib destiné à être utilisé selon la revendication 11 ou la revendication 12, dans lequel le lonafamib est administré par voie orale à une dose de 50 mg deux fois par jour et le ritonavir est administré par voie orale à une dose de 100 mg deux fois par jour.

14. Lonafarnib destiné à être utilisé selon l'une quelconque des revendications 10 à 13, dans lequel le lonafarnib et le ritonavir sont administrés au patient comme une thérapie combinée pendant au moins 90 jours.

15. Lonafarnib destiné à être utilisé selon l'une quelconque des revendications 10 à 14, dans lequel les lonafarnib et ritonavir sont administrés comme (i) des formes galéniques unitaires de lonafarnib et de ritonavir qui sont administrées au même moment environ ; ou (ii) une forme galénique unitaire unique.

16. Lonafarnib destiné à être utilisé selon l'une quelconque des revendications 10 à 15, dans lequel le patient reçoit aussi l'administration d'interféron, préférablement dans lequel l'interféron est l'interféron alpha ou l'interféron lambda, plus préférablement dans lequel l'interféron alpha est l'interféron pégylé alpha-2a.

17. Lonafarnib destiné à être utilisé selon la revendication 16, dans lequel l'interféron est l'interféron lambda ou l'interféron pégylé alpha-2a et est administré à une dose de 180 µg une fois par semaine.

18. Lonafarnib destiné à être utilisé selon la revendication 16, dans lequel l'interféron est l'interféron lambda et est administré à une dose de 120 µg une fois par semaine.
